(19) Europäisches Patentamt
European Patent Office
Office européen des brevets

(11) Veröffentlichungsnummer: **0 337 211**
**B1**

(12) # EUROPÄISCHE PATENTSCHRIFT

(45) Veröffentlichungstag der Patentschrift: **11.07.90**

(51) Int. Cl.⁵: **C07C 69/732,** C07C 69/738, A01N 37/36

(21) Anmeldenummer: **89105691.3**

(22) Anmeldetag: **31.03.89**

(54) **Ortho-substituierte Carbonsäurebenzylester und diese enthaltende Fungizide.**

(30) Priorität: **12.04.88 DE 3812082**

(43) Veröffentlichungstag der Anmeldung: **18.10.89 Patentblatt 89/42**

(45) Bekanntmachung des Hinweises auf die Patenterteilung: **11.07.90 Patentblatt 90/28**

(84) Benannte Vertragsstaaten: **AT BE CH DE ES FR GB GR IT LI NL SE**

(56) Entgegenhaltungen:
**EP-A-17 882 6**
**EP-A-20 360 8**
**EP-A-22 691 7**
**GB-A- 2 202 844**

(73) Patentinhaber: **BASF Aktiengesellschaft, Carl-Bosch-Strasse 38, D-6700 Ludwigshafen(DE)**

(72) Erfinder: **Wenderoth, Bernd, Dr., Schwalbenstrasse 26, D-6840 Lampertheim(DE)**
Erfinder: **Brand, Siegbert, Dr., Hegelstrasse 39, D-6940 Weinheim(DE)**
Erfinder: **Schuetz, Franz, Dr., Budapester Strasse 45, D-6700 Ludwigshafen(DE)**
Erfinder: **Sauter, Hubert, Dr., Neckarpromenade 20, D-6800 Mannheim 1(DE)**
Erfinder: **Ammermann, Eberhard, Dr., Sachsenstrasse 3, D-6700 Ludwigshafen(DE)**
Erfinder: **Lorenz, Gisela, Dr., Erlenweg 13, D-6730 Neustadt(DE)**

**EP 0 337 211 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue ortho-substituierte Carbonsäurebenzylester, ihre Herstellung und ihre Verwendung als Fungizide.

Es ist bekannt, N-Tridecyl-2,6-dimethylmorpholin oder seine Salze, z.B. das Acetat, als Fungizide zu verwenden (DE 1 164 152, 1 173 722). Ihre Wirkung ist jedoch in manchen Fällen ungenügend. Es ist ferner bekannt, α-[(2-Phenoxymethylen)phenyl]- oder α-[(2-alkoxymethylen)-phenyl]-β-methoxyacrylsäuremethylester als Fungizide zu verwenden (DE-35 45 319.2, DE-36 20 860.4). Ihre Wirkung ist jedoch unbefriedigend.

Es wurde nun gefunden, daß neue ortho-substituierte Carbonsäurebenzylester der Formel I

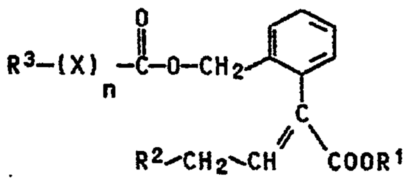

(I)

in der

$R^1$ $C_1$-$C_5$-Alkyl,

$R^2$ Wasserstoff, $C_1$-$C_4$-Alkyl, $C_1$-$C_4$-Alkoxy

$R^3$ Wasserstoff, Halogen, Cyano, Aryl, Aryloxy, wobei der aromatische Ring gegebenenfalls durch einen oder mehrere der folgenden Reste substituiert ist:

$C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl, Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ Heteroaryl, Adamantyl, Fluorenyl oder einen $C_3$-$C_7$-Cycloalkylrest oder $C_5$-$C_6$-Cycloalkenylrest bedeutet, wobei die Reste gegebenenfalls substituiert sind durch $C_1$-$C_4$-Alkyl, Methyl, Ethyl, Halogen (Chlor, Brom), $C_1$-$C_2$-Halogenalkyl (Trifluormethyl, Tetrabromethyl, Dichlor-dibromethyl), $C_3$-$C_4$-Alkenyl (Methylvinyl, Dimethylvinyl), $C_2$-$C_4$-Halogenalkenyl, (Dichlorvinyl, Dichlorbutadienyl, Difluorvinyl, Trifluormethylvinyl), Methoxycarbonyl-$C_3$-$C_4$-Alkenyl (Methyl-Methoxycarbonylvinyl), Cyclopentylidenmethyl, Halogenphenyl (Chlorphenyl), $C_1$-$C_2$-Alkoxyphenyl (Ethoxyphenyl), $C_1$-$C_4$-Alkylphenyl (tert. Butylphenyl),

X einen geradkettigen oder verzweigten, gegebenenfalls durch Halogen oder Hydroxy sustituierten, gegebenenfalls ungesättigten $C_1$-$C_{12}$-Alkylenrest bedeutet und

n die Zahlen 0 oder 1 bedeutet,

eine ausgezeichnete fungizide Wirkung haben.

Die in der allgemeinen Formel I aufgeführten Reste können beispielsweise folgende Bedeutung haben:

$R^1$ steht z.B. für $C_1$-$C_3$-Alkyl, Methyl, Ethyl, i-Propyl und

$R^2$ kann z.B. Wasserstoff, Methyl, Ethyl, Propyl, Butyl, Methoxy, Ethoxy, Isopropoxy oder Butoxy sein.

$R^3$ kann z.B. Wasserstoff, Halogen (z.B. Fluor, Chlor, Brom), Cyano, Aryl (Phenyl, Naphthyl) oder Aryloxy (Phenyloxy) sein, wobei der aromatische Ring gegebenenfalls durch einen oder mehrere der folgenden Reste substituiert ist:

$C_1$-$C_6$-Alkyl (z.B. Methyl, Ethyl, n- oder iso-Propyl, n-, iso-, sec.- oder tert.-Butyl, n-, iso-, sec.-, tert.- oder neo-Pentyl, Hexyl), $C_2$-$C_4$-Alkenyl (z.B. Vinyl, Allyl), $C_1$-$C_2$-Halogenalkyl (z.B. Difluormethyl, Trifluormethyl), $C_1$-$C_6$-Alkoxy (z.B. Methoxy, Ethoxy, iso-Propoxy, tert.-Butoxy), $C_1$-$C_4$-Alkoxy-$C_1$-$C_4$-alkyl (z.B. Methoxymethyl), Aryl-$C_1$-$C_2$-alkyl (z.B. Benzyl), Aryloxy (z.B. Phenoxy), Aryloxy-$C_1$-$C_4$-alkyl (z.B. Phenoxymethyl, Phenoxyethyl), Aryloxy-$C_1$-$C_4$-alkoxy, Halogenaryloxy-$C_1$-$C_4$-alkoxy, (z.B. Phenoxymethoxy, Phenoxyethoxy, Phenoxypropoxy, 2-Chlor-phenoxy-ethoxy, 4-Chlor-phenoxy-ethoxy), Halogen (z.B. Fluor, Chlor, Brom, Jod), Halogen-$C_1$-$C_4$-alkoxy (z.B. 1,1,2,2-Tetrafluorethoxy), $C_1$-$C_4$-Alkylthio (z.B. Methylthio), Thiocyanato, Cyano, Nitro.

$R^3$ kann ferner bedeuten:

Heteroaryl (z.B. Furyl, Pyrrolyl), Piperidinyl, Morpholinyl, $C_3$-$C_7$-Cycloalkyl, $C_5$-$C_6$-Cycloalkenyl, (z.B. Cyclopropyl, Cyclobutyl, Cyclopentyl, Cyclopentenyl, Cyclohexyl, Cyclohexenyl, Cycloheptyl), 1-Adamantyl, 9-Fluorenyl oder einen substituierten Cyclohexylrest (1-Methylcyclohexyl)- oder einen Cyclopropylrest, der substituiert ist durch $C_1$-$C_4$-Alkyl (z.B. Methyl, Ethyl), Halogen (Chlor, Brom), $C_1$-$C_2$-Halogenalkyl (Trifluormethyl, Tetrabromethyl, Dichlor-dibromethyl), $C_3$-$C_4$-Alkenyl (Methylvinyl, Dimethylvinyl), $C_2$-$C_4$-Halogenalkenyl (Dichlorvinyl, Dichlorbutadienyl, Difluorvinyl, Trifluormethylvinyl), Methoxycarbonyl-$C_3$-$C_4$-Alkenyl (Methyl-Methoxycarbonylvinyl), Cyclopentylidenmethyl, Halogenphenyl (Chlorphenyl), $C_1$-$C_2$-Alkoxyphenyl (Ethoxyphenyl), $C_1$-$C_4$-Alkylphenyl (tert. Butylphenyl), wie zum Beispiel:

2,2-Dimethyl-3-(2′,2′-dimethylvinyl)-cyclopropyl (A1), 2,2-Dimethyl-3-(2′,2′-dichlorvinyl)-cyclopropyl (A2), 2,2-Dimethyl-3-(2′,2′-dibromvinyl)-cyclopropyl (A3), 2,2-Dimethyl-3-(2′-trifluormethyl-2′-chlorvinyl)-cyclopropyl (A4), 2,2-Dichlor-3,3-dimethyl-cyclopropyl (A5), 2,2,3,3-Tetramethyl-cyclopropyl

(A6), 2,2-Dimethyl-3-(2',2'-difluorvinyl)-cyclopropyl (A7), 2,2-Dimethyl-3-(2'-trifluormethyl-2'fluorvinyl)-cyclopropyl (A8), 2,2-Dimethyl-3-(2'-methyl-2'-methoxycarbonylvinyl)-cyclo-propyl (A9), 2,2-Dimethyl-3-(4',4'-dichlorbutadienyl)-cyclopropyl (A10), 2,2-Dimethyl-3-(1'-brom-2',2',2'-tribromethyl)-cyclopropyl (A11), 2,2-Dimethyl-3-(1'-brom-2',2'-dichlor-2'-bromethyl)-cyclopropyl (A12), 1-(2',4'-Dichlorphenyl)-cyclopropyl (A13), 1-(4'-Chlorphenyl)-cyclopropyl (A14), 1-(4'-Ethoxyphenyl)-2,2-dichlorcyclopropyl (A15), 2,2-Dimethyl-3-(4'-tert.-Butylphenyl)-cyclopropyl (A16), 1-Methyl-2,2-dichlorcyclopropyl (A17),

Xkann beispielsweise bedeuten:

einen geradkettigen $C_1$-$C_{12}$-Alkylenrest (z.B. Methylen, Ethylen, Propylen, Butylen, Pentylen, Hexylen, Heptylen), einen verzweigten $C_1$-$C_{12}$-Alkylenrest (z.B. Methylmethylen, Dimethylmethylen, Ethylmethylen, n- oder iso-Propylmethylen, Methylethylen, Methylpropylen, Dimethylpropylen, Ethylpropylen, Methylbutylen, Dimethylbutylen, Ethylbutylen, n- oder iso-Propylbutylen, Methylpentylen, Dimethylpentylen, Trimethylpentylen, Methylhexylen, Dimethylhexylen, Trimethylhexylen, Ethylhexylen, n- oder iso-Propylhexylen, Methylheptylen), einen $C_2$-$C_8$-Alkenylenrest (z.B. Vinylen, Allylen, Methylallylen, Butenylen, Methylbutenylen), einen durch Halogen substituierten $C_1$-$C_{12}$-Alkylenrest (z.B. Chlormethylen, Dichlormethylen, Fluormethylen, Difluormethylen, Brommethylen, Dibrommethylen, Chlorethylen, Fluorethylen, Bromethylen, Fluorpropylen, Chlorpropylen, Brompropylen, Fluorbutylen, Chlorbutylen, Brombutylen), einen durch Halogen substituierten $C_2$-$C_4$-Alkenylenrest (z.B. Chlorvinylen, Dichlorvinylen), einen durch Hydroxy substituierten $C_1$-$C_8$-Alkylenrest (z.B. Hydroxymethylen, Hydroxyethylen).

$X_n$bedeutet für den Fall n = 0 eine Einfachbindung.

Die neuen Verbindungen der Formel I fallen bei ihrer Herstellung aufgrund der C=C-Doppelbindung als E/Z-Isomerengemische an, die in üblicher Weise, z.B. durch Kristallisation oder Chromatographie, in die einzelnen Komponenten getrennt werden können. Sowohl die einzelnen isomeren Verbindungen als auch ihre Gemische werden von der Erfindung umfaßt und sind als Fungizide brauchbar.

Die neuen Verbindungen der Formel I können z.B. hergestellt werden, indem man ein orthosubstituiertes Benzylbromid der allgemeinen Formel III, in der $R^1$ und $R^2$ die oben angegebene Bedeutung haben, mit einem Alkali-, Erdalkali- oder Ammoniumsalz einer Carbonsäure der Formel II, worin $R^3$, X und n die oben angegebene Bedeutung haben, in einem Lösungs- oder Verdünnungsmittel und gegebenenfalls unter Zusatz eines Katalysators zu den neuen Verbindungen umsetzt.

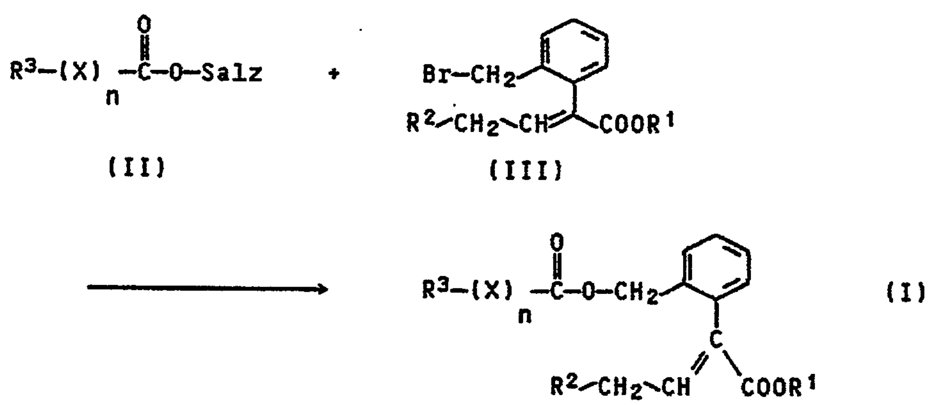
(II) (III) (I)

Die Herstellung von Carbonsäureestern aus Alkylhalogeniden und Carboxylaten ist bekannt (vgl. z.B. Synthesis 1975, 805).

Als Lösungs- oder Verdünnungsmittel für die Reaktion von II mit III kommen z.B. Aceton, Acetonitril, Dimethylsulfoxid, Dioxan, Dimethylformamid, N-Methylpyrrolidon, N,N'-Dimethylpropylenharnstoff oder Pyridin in Betracht.

Außerdem kann es von Vorteil sein, der Reaktionsmischung einen Katalysator, wie z.B. Kaliumiodid oder Tetramethylethylendiamin, in einer Menge von 0,01 bis 10 % (Gew.%), bezogen auf Verbindung III, zuzusetzen.

Die entsprechenden Umsetzungen können auch im Zweiphasensystem (z.B. Tetrachlorkohlenstoff/Wasser) durchgeführt werden. Als Phasentransferkatalysatoren kommen z.B. Trioctylpropylammoniumchlorid oder Cetyltrimethylammoniumchlorid in Betracht (vgl. Synthesis 1974, 867).

Die Carboxylate der Formel II sind bekannt. Sie können aus den entsprechenden Carbonsäuren mit Basen (z.B. Kaliumhydroxid) in einem inerten Lösungsmittel (z.B. Ethanol) hergestellt werden.

Die ortho-substituierten Benzylbromide der Formel III lassen sich herstellen, indem man bekannte α-Ketocarbonsäureester der Formel IV (vgl. z.B. J.M. Photis, Tetrahedron Lett. 1980, 3539)

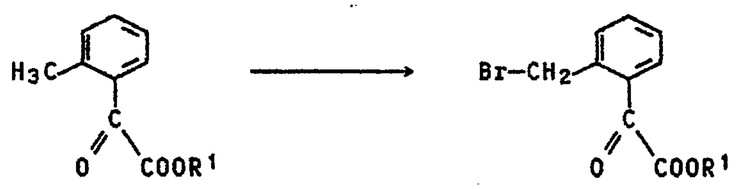

(IV) (V)

mit Brom in einem Lösungsmittel wie zum Beispiel Tetrachlormethan, gegebenenfalls unter Belichtung mit einer Lichtquelle (z.B. Hg-Dampf-Lampe, 300 W), oder mit N-Bromsuccinimid (Horner, Winkelmann, Angew. Chem. 71, 349 (1959) zu den α-Ketocarbonsäureestern der allgemeinen Formel V umsetzt, wobei $R^1$ die obengenannten Bedeutungen hat.

Die α-Ketocarbonsäureester der Formel V lassen sich in einer Wittig-Reaktion mit einem Alkyl- oder Alkoxymethyltriphenyl-phosphoniumbromid in Gegenwart einer Base wie z. B. n-Butyllithium, Natriummethylat, Kalium-tert.-butylat oder Natriumhydrid (vgl. G. Wittig, U. Schöllkopf, Org. Synth., Coll. Vol. V, 751-54 (1973)) in die obengenannten ortho-substituierten Benzylbromide der allgemeinen Formel III überführen.

Die neuen Verbindungen der Formel I können zum Beispiel auch in der Weise hergestellt werden, daß man die neuen α-Ketocarbonsäureester der allgemeinen Formel VI in einer Wittig-Reaktion mit einem Alkyl- oder Alkoxymethyl-triphenylphosphoniumbromid in Gegenwart einer Base wie z.B. n-Butyllithium, Natriummethylat, Kalium-tert.-butylat oder Natriumhydrid umsetzt, wobei $R^1$, $R^2$, $R^3$, X und n die obengenannten Bedeutungen haben:

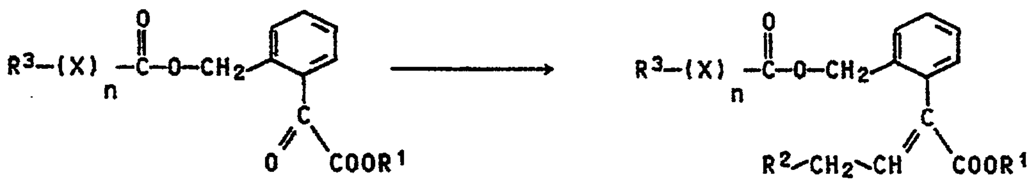

(VI) (I)

Die neuen α-Ketocarbonsäureester der Formel VI sind wertvolle Zwischenprodukte. Sie können zum Beispiel hergestellt werden, indem man die obengenannte Verbindung der Formel V mit einem Alkali-, Erdalkali- oder Ammoniumsalz einer Carbonsäure der Formel II, worin $R^3$, $R^1$, X und n die oben angegebenen Bedeutungen haben, in einem Lösungs- oder Verdünnungsmittel und gegebenenfalls unter Zusatz eines Katalysators zu den neuen Verbindungen der Formel VI umsetzt.

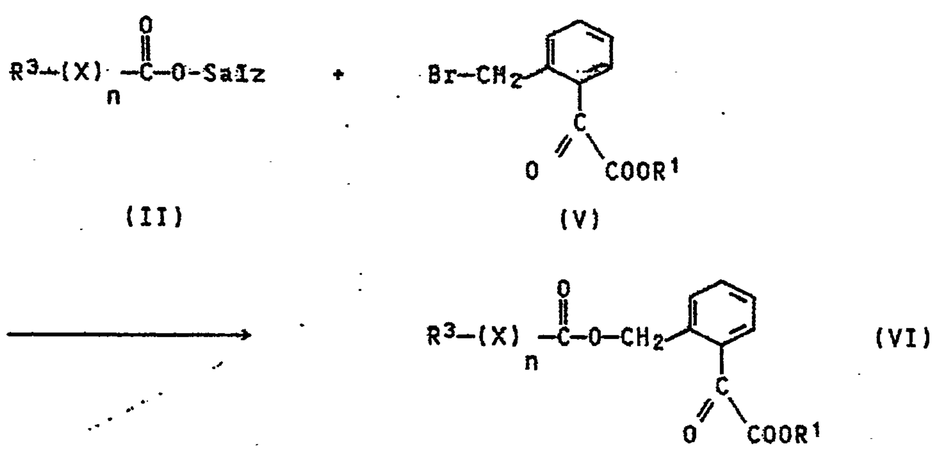

(II) (V)

(VI)

Die Herstellung von Carbonsäureestern aus Alkylhalogeniden und Carboxylaten ist bekannt (vgl. z. B. Synthesis 1975, 805).

Als Lösungs- oder Verdünnungsmittel für die Reaktion von II mit V kommen Aceton, Acetonitril, Dimethylsulfoxid, Dioxan, Dimethylformamid, N-Methylpyrrolidon, N,N'-Dimethylpropylenharnstoff oder Pyridin in Betracht.

Außerdem kann es von Vorteil sein, der Reaktionsmischung einen Katalysator, wie z. B. Kaliumiodid oder Tetramethylethylendiamin, in einer Menge von 0,01 bis 10 % (Gew.%), bezogen auf Verbindung V zuzusetzen.

Die entsprechenden Umsetzungen können auch im Zweiphasensystem (z.B. Tetrachlorkohlenstoff/Wasser) durchgeführt werden. Als Phasentransferkatalysatoren kommen z. B. Trioctylpropylammoniumchlorid oder Cetyltrimethylammoniumchlorid in Betracht (vgl. Synthesis 1974, 867).

Die Herstellung der neuen Verbindungen der Formeln I und VI wird durch folgende Beispiele erläutert.

Vorschrift 1

Herstellung von 2-(Brommethyl)-phenylglyoxylsäuremethylester

5,34 g (30 mmol) 2-Methylphenylglyoxylsäuremethylester und 5,34 g (30 mmol) N-Bromsuccinimid werden in 1000 ml Tetrachlormethan für eine Stunde mit einer 300 W-Hg-Dampf-Lampe bestrahlt. Dann wird die organische Phase 1x mit Wasser und 3x mit Natriumhydrogencarbonat-Lösung gewaschen und über Natriumsulfat/Natriumcarbonat getrocknet. Nach dem Einengen wird das Rohprodukt an Kieselgel mit Methyl-t.-Butylether/n-Hexan (1/9) chromatographiert. Man erhält 3,8 g (49 %) der obengenannten Verbindung als gelbes Öl.

$^1$H-NMR ($CDCl_3$): δ = 3,97 (S, 3H), 4,90 (S, 2H), 7,4-7,8 (m, 4H)
IR (Film): 2955, 1740, 1689, 1435, 1318, 1207, 999 $cm^{-1}$

Vorschrift 2

Herstellung von ortho-[2,2-Dimethyl-3-(2′,2′-dichlorvinyl)-cyclo-propylcarboxymethylen]-phenylglyoxylsäuremethylester

53,4 g (0,3 mol) 2-Methylphenylglyoxylsäuremethylester und 58,7 g (0,33 mol) N-Bromsuccinimid werden in 3000 ml Tetrachlormethan für 1,5 Stunden mit einer 300 W-Hg-Dampf-Lampe bestrahlt. Dann wird die Lösung auf ein Drittel eingeengt, mit Wasser gewaschen, über $Na_2SO_4$ getrocknet und eingeengt. Der Rückstand wird in 500 ml N-Methyl-2-pyrrolidon gelöst, mit 49,4 g (0,2 mol) 2,2-Dimethyl-3(2′,2′-dichlorvinyl)-cyclopropylcarbonsäure-Kaliumsalz und einer Spatelspitze Kaliumjodid versetzt und 16 Stunden bei Raumtemperatur (21°C) gerührt. Dann wird auf Wasser gegossen und mit Methyl-tert.-butylether gründlich extrahiert. Die vereinigten organischen Phasen werden mit Wasser gewaschen, über Natriumsulfat getrocknet und eingeengt. Das Rohprodukt wird über eine Kieselgel-Säule (Hexan/Methyl-tert.-butylether = 5/1) chromatographiert. Man erhält 40 g (52 %) des obengenannten Esters als Öl (cis/trans-Isomere am Dreiring).

$^1$H-NMR ($CDCl_3$): δ = 1,22/1,30 (2 S,3 H), 1,31/1,35 (2S,3 H), 1,70/1,95 (2d, 1H), 2.10/2,28 (2t, 1H), 4,0 (S, 3H), 5,48/5,51 (2S, 1H), 5,65/6,25 (2d, 1H), 7,4-7,8 (m, 4H).

Beispiel 1

Herstellung von α-[ortho-(2,2-Dimethyl-3-(2′,2′-dichlorvinyl)-cyclopropylcarboxy-methylen)-phenyl]-β-methylacrylsäuremethylester (Verbindung Nr. 286)

11,3 g (27 mmol) Ethyltriphenylphosphoniumbromid werden unter Stickstoff in 100 ml absolutem Tetrahydrofuran vorgelegt. Bei 0°C werden 10,8 ml (27 mmol) einer 2,5 molaren Lösung von n-Butylithium in Hexan zugetropft. Nach einer Stunde Rühren bei 0°C werden bei -78°C 8 g (21 mmol) in 40 ml absolutem Tetrahydrofuran gelöster ortho-[2,2-Dimethyl-3-(2′,2′-dichlorvinyl)-cyclopropylcarboxymethylen]-phenylglyoxylsäuremethylester zugetropft. Man erwärmt langsam bis auf Raumtemperatur (21°C) und gießt auf gesättigte Ammoniumchloridlösung. Dann wird mit Methyl-tert.-butylether mehrmals extrahiert, mit Wasser gewaschen, über Natriumsulfat getrocknet und eingeengt. Das Rohprodukt wird über eine Kieselgel-Säule (Methyl-tert.-butylether/n-Hexan = 1/2) chromatographisch gereinigt. Man erhält 4 g (48 %) des obengenannten Esters als Öl (cis/trans-Isomere am Dreiring).

$^{13}$C-NMR ($CDCl_3$): δ = 14.99/20.13, 15.37, 22.59/28.40, 27.40/28.90, 31.89/34.91, 32.67/32.98, 51.90, 64.31/64.59, 121.03/122.93, 124.93/126.97, 128.09, 128.25, 128.93, 130.31, 133.56, 134.75, 135.11, 141.25, 166.98, 170.07/170.67.

In entsprechender Weise können die in der folgenden Tabelle aufgeführten Verbindungen hergestellt werden.

Tabelle 1

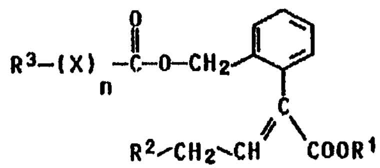

(I)

| Nr. | $R^3$ | $(X)_n$ | $R^2$ | $R^1$ | Fp (°C) | IR ($cm^{-1}$) |
|---|---|---|---|---|---|---|
| 1 | H | $-CH_2-$ | H | $CH_3$ | | |
| 2 | H | $-CH_2-CH_2-$ | H | $CH_3$ | | |
| 3 | H | $-CH_2-CH(CH_3)-$ | H | $CH_3$ | | |
| 4 | H | $-CH_2-C(CH_3)_2-$ | H | $CH_3$ | Öl | 1721, 1253, 1149, 760 |
| 5 | H | $-CH=CH-$ | H | $CH_3$ | | |
| 6 | H | $-CH=C(CH_3)-$ | H | $CH_3$ | | |
| 7 | H | $-C \equiv C-$ | H | $CH_3$ | | |
| 8 | H | $-CH_2-CH_2-CH_2-$ | H | $CH_3$ | | |
| 9 | H | $-CH_2-CH_2-CH(CH_3)-$ | H | $CH_3$ | | |
| 10 | H | $-CH_2-CH(CH_3)-CH_2-$ | H | $CH_3$ | | |
| 11 | H | $-CH_2-CH_2-C(CH_3)_2-$ | H | $CH_3$ | | |
| 12 | H | $-CH_2-C(CH_3)_2-CH_2-$ | H | $CH_3$ | | |
| 13 | H | $-CH_2-CH_2-C(C_2H_5)_2-$ | H | $CH_3$ | | |
| 14 | H | $-CH=CH-CH_2-$ | H | $CH_3$ | | |
| 15 | H | $-CH_2-CH=CH-$ | H | $CH_3$ | | |
| 16 | H | $-CH_2-C(CH_3)=CH-$ | H | $CH_3$ | | |
| 17 | H | $-CH_2-CH=C(CH_3)-$ | H | $CH_3$ | | |
| 18 | H | $-(CH_2)_4-$ | H | $CH_3$ | | |
| 19 | H | $-CH_2-CH_2-CH_2-CH(CH_3)-$ | H | $CH_3$ | | |
| 20 | H | $-CH_2-CH(CH_3)-CH_2-CH_2-$ | H | $CH_3$ | | |
| 21 | H | $-(CH_2)_3-C(CH_3)_2-$ | H | $CH_3$ | | |
| 22 | H | $-(CH_2)_3-CH(C_2H_5)-$ | H | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 23 | H | $-(CH_2)_3-CH(n-C_3H_7)-$ | H | $CH_3$ | | |
| 24 | H | $-CH_2-CH=CH-CH_2-$ | H | $CH_3$ | | |
| 25 | H | $-CH_2-C(CH_3)=CH-CH_2-$ | H | $CH_3$ | | |
| 26 | H | $-(CH_2)_5-$ | H | $CH_3$ | | |
| 27 | H | $-(CH_2)_4-CH(CH_3)-$ | H | $CH_3$ | | |
| 28 | H | $-(CH_2)_4-CH(C_2H_5)-$ | H | $CH_3$ | | |
| 29 | H | $-(CH_2)_3-CH(CH_3)-CH_2-$ | H | $CH_3$ | | |
| 30 | H | $-CH_2-CH=CH-CH=CH-$ | H | $CH_3$ | | |
| 31 | H | $-CH_2-C(CH_3)=CH-CH=CH-$ | H | $CH_3$ | | |
| 32 | H | $-(CH_2)_6-$ | H | $CH_3$ | | |
| 33 | H | $-(CH_2)_5-CH(CH_3)-$ | H | $CH_3$ | | |
| 34 | H | $-(CH_2)_4-CH(CH_3)-CH_2-$ | H | $CH_3$ | | |
| 35 | H | $-(CH_2)_5-CH(n-C_3H_7)-$ | H | $CH_3$ | | |
| 36 | H | $-(CH_2)_7-$ | H | $CH_3$ | | |
| 37 | H | $-(CH_2)_6-CH(CH_3)-$ | H | $CH_3$ | | |
| 38 | H | $-(CH_2)_5-CH(CH_3)-CH_2-$ | H | $CH_3$ | | |
| 39 | H | $-(CH_2)_6-C(CH_3)_2-$ | H | $CH_3$ | | |
| 40 | H | $-(CH_2)_8-$ | H | $CH_3$ | | |
| 41 | H | $-(CH_2)_9-$ | H | $CH_3$ | | |
| 42 | H | $-(CH_2)_{10}-$ | H | $CH_3$ | | |
| 43 | H | $-CHCl-$ | H | $CH_3$ | | |
| 44 | H | $-CCl_2-$ | H | $CH_3$ | | |
| 45 | Cl | $-CCl_2-$ | H | $CH_3$ | | |
| 46 | H | $-CHBr-$ | H | $CH_3$ | | |
| 47 | H | $-CBr_2-$ | H | $CH_3$ | | |
| 48 | Br | $-CBr_2$ | H | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 49 | H | $-CHF-$ | H | $CH_3$ | | |
| 50 | H | $-CF_2-$ | H | $CH_3$ | | |
| 51 | F | $-CF_2-$ | H | $CH_3$ | | |
| 52 | H | $-CH=CCl-$ | H | $CH_3$ | | |
| 53 | H | $-CCl=CCl-$ | H | $CH_3$ | | |
| 54 | Cl | $-C(CH_3)_2-$ | H | $CH_3$ | | |
| 55 | H | $-C(CH_3)_2-$ | H | $CH_3$ | | |
| 56 | H | $-CHCl-CH(CH_3)-$ | H | $CH_3$ | | |
| 57 | H | $-CHCl-C(CH_3)_2-$ | H | $CH_3$ | | |
| 58 | H | $-CHBr-CH(CH_3)-$ | H | $CH_3$ | | |
| 59 | Br | $-C(C_2H_5)_2-$ | H | $CH_3$ | | |
| 60 | H | $-CH(OH)-$ | H | $CH_3$ | | |
| 61 | H | $-CH_2-CH(OH)-$ | H | $CH_3$ | | |
| 62 | H | $-CH_2-CH_2-CH(OH)-$ | H | $CH_3$ | | |
| 63 | H | $-CH_2-CH(OH)-CH_2-$ | H | $CH_3$ | | |
| 64 | H | $-CH(OH)-CH_2-$ | H | $CH_3$ | | |
| 65 | H | $-CH(OH)-C(CH_3)_2-$ | H | $CH_3$ | | |
| 66 | H | $-CH_2-C(OH)(CH_3)-$ | H | $CH_3$ | | |
| 67 | H | $-CH_2-CH(CH_3)-CH(OH)-$ | H | $CH_3$ | | |
| 68 | H | $-CH=CH-CH(OH)-$ | H | $CH_3$ | | |
| 69 | H | $-CH=CH-CH_2-CH(OH)-$ | H | $CH_3$ | | |
| 70 | CN | $-CH_2-$ | H | $CH_3$ | | |
| 71 | Cyclopropyl | - | H | $CH_3$ | Öl | 1721, 1390, 1256, 1170, 760 |
| 72 | Cyclobutyl | - | H | $CH_3$ | | |
| 73 | Cyclopentyl | - | H | $CH_3$ | | |
| 74 | Cyclohexyl | - | H | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 75 | Adamantyl | - | H | $CH_3$ | | |
| 76 | 9-Fluorenyl | - | H | $CH_3$ | | |
| 77 | Cyclopentyl | $-CH_2-$ | H | $CH_3$ | | |
| 78 | 3-Cyclopentenyl | $-CH_2-$ | H | $CH_3$ | | |
| 79 | Cyclohexyl | $-CH_2-$ | H | $CH_3$ | | |
| 80 | Cyclopentyl | $-CH_2-CH_2-$ | H | $CH_3$ | | |
| 81 | Cyclohexyl | $-CH_2-CH_2-$ | H | $CH_3$ | | |
| 82 | Cyclohexyl | $-(CH_2)_3-$ | H | $CH_3$ | | |
| 83 | $C_6H_5$ (=Phenyl) | - | H | $CH_3$ | Öl | 1719, 1271, 713 |
| 84 | $2-CH_3-C_6H_4$ | - | H | $CH_3$ | | |
| 85 | $3-CH_3-C_6H_4$ | - | H | $CH_3$ | | |
| 86 | $4-CH_3-C_6H_4$ | - | H | $CH_3$ | | |
| 87 | $2,3-(CH_3)_2-C_6H_3$ | - | H | $CH_3$ | | |
| 88 | $2,4-(CH_3)_2-C_6H_3$ | - | H | $CH_3$ | | |
| 89 | $2,6-(CH_3)_2-C_6H_3$ | - | H | $CH_3$ | | |
| 90 | $3,4-(CH_3)_2-C_6H_3$ | - | H | $CH_3$ | | |
| 91 | $3,5-(CH_3)_2-C_6H_3$ | - | H | $CH_3$ | | |
| 92 | $2,4,6-(CH_3)_2-C_6H_2$ | - | H | $CH_3$ | | |
| 93 | $4-t-C_4H_9-C_6H_4$ | - | H | $CH_3$ | | |
| 94 | $2-C_6H_5-C_6H_4$ | - | H | $CH_3$ | | |
| 95 | $4-C_6H_5-C_6H_4$ | - | H | $CH_3$ | | |
| 96 | 2-Benzyl-$C_6H_4$ | - | H | $CH_3$ | | |
| 97 | 4-Benzyl-$C_6H_4$ | - | H | $CH_3$ | | |
| 98 | $2-Cl-C_6H_4$ | - | H | $CH_3$ | | |
| 99 | $3-Cl-C_6H_4$ | - | H | $CH_3$ | | |
| 100 | $4-Cl-C_6H_4$ | - | H | $CH_3$ | | |

Tabelle 1 (Fortsetzung)

| Nr. | $R^3$ | $(X)_n$ | $R^2$ | $R^1$ | Fp ($^0$C) | IR ($cm^{-1}$) |
|---|---|---|---|---|---|---|
| 101 | 2,4-$Cl_2$-$C_6H_3$ | - | H | $CH_3$ | | |
| 102 | 2,5-$Cl_2$-$C_6H_3$ | - | H | $CH_3$ | | |
| 103 | 2,6-$Cl_2$-$C_6H_3$ | - | H | $CH_3$ | | |
| 104 | 3,4-$Cl_2$-$C_6H_3$ | - | H | $CH_3$ | | |
| 105 | 3,5-$Cl_2$-$C_6H_3$ | - | H | $CH_3$ | | |
| 106 | 2,4,5-$Cl_3$-$C_6H_2$ | - | H | $CH_3$ | | |
| 107 | 2,3,4,5,6-$Cl_5$-$C_6$ | - | H | $CH_3$ | | |
| 108 | 2-F,4-Cl-$C_6H_3$ | - | H | $CH_3$ | | |
| 109 | 2-F-$C_6H_4$ | - | H | $CH_3$ | | |
| 110 | 3-F-$C_6H_4$ | - | H | $CH_3$ | | |
| 111 | 4-F-$C_6H_4$ | - | H | $CH_3$ | | |
| 112 | 2,4-$F_2$-$C_6H_3$ | - | H | $CH_3$ | | |
| 113 | 2,6-$F_2$-$C_6H_3$ | - | H | $CH_3$ | | |
| 114 | 2,3,4,5,6-$F_5$-$C_6$ | - | H | $CH_3$ | | |
| 115 | 2-$CF_3$-$C_6H_4$ | - | H | $CH_3$ | | |
| 116 | 3-$CF_3$-$C_6H_4$ | - | H | $CH_3$ | | |
| 117 | 4-$CF_3$-$C_6H_4$ | - | H | $CH_3$ | | |
| 118 | 2-$OCH_3$-$C_6H_4$ | - | H | $CH_3$ | | |
| 119 | 3-$OCH_3$-$C_6H_4$ | - | H | $CH_3$ | | |
| 120 | 4-$OCH_3$-$C_6H_4$ | - | H | $CH_3$ | | |
| 121 | 2-Phenoxy-$C_6H_4$ | - | H | $CH_3$ | | |
| 122 | 3-Phenoxy-$C_6H_4$ | - | H | $CH_3$ | | |
| 123 | 4-Phenoxy-$C_6H_4$ | - | H | $CH_3$ | | |
| 124 | 4-Ethoxy-$C_6H_4$ | - | H | $CH_3$ | | |
| 125 | 2-Phenoxyethoxy-$C_6H_4$ | - | H | $CH_3$ | | |
| 126 | 2-(2'-Cl-Phenoxyethoxy)-$C_6H_4$ | - | H | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 127 | 2-(3'-Cl-Phenoxyethoxy)-$C_6H_4$ | - | H | $CH_3$ | | |
| 128 | 2-(4'-Cl-Phenoxyethoxy)-$C_6H_4$ | - | H | $CH_3$ | | |
| 129 | 3-Phenoxyethoxy-$C_6H_4$ | - | H | $CH_3$ | | |
| 130 | 3-(4'-Cl-Phenoxyethoxy)-$C_6H_4$ | - | H | $CH_3$ | | |
| 131 | 4-Phenoxyethoxy-$C_6H_4$ | - | H | $CH_3$ | | |
| 132 | 2-Phenoxypropoxy-$C_6H_4$ | - | H | $CH_3$ | | |
| 133 | 3-Phenoxypropoxy-$C_6H_4$ | - | H | $CH_3$ | | |
| 134 | 4-Phenoxypropoxy-$C_6H_4$ | - | H | $CH_3$ | | |
| 135 | $C_6H_5$ | $-CH_2-$ | H | $CH_3$ | | |
| 136 | 2-$CH_3$-$C_6H_4$ | $-CH_2-$ | H | $CH_3$ | | |
| 137 | $C_6H_5$ | $-CH(CH_3)-$ | H | $CH_3$ | | |
| 138 | 4-Phenyl-$C_6H_4$ | $-CH_2-$ | H | $CH_3$ | | |
| 139 | 2-F-$C_6H_4$ | $-CH_2-$ | H | $CH_3$ | | |
| 140 | 3-F-$C_6H_4$ | $-CH_2-$ | H | $CH_3$ | | |
| 141 | 4-F-$C_6H_4$ | $-CH_2-$ | H | $CH_3$ | | |
| 142 | 2-Cl-$C_6H_4$ | $-CH_2-$ | H | $CH_3$ | | |
| 143 | 3-Cl-$C_6H_4$ | $-CH_2-$ | H | $CH_3$ | | |
| 144 | 4-Cl-$C_6H_4$ | $-CH_2-$ | H | $CH_3$ | | |
| 145 | 2,4-$Cl_2$-$C_6H_3$ | $-CH_2-$ | H | $CH_3$ | | |
| 146 | 2,6-$Cl_2$-$C_6H_3$ | $-CH_2-$ | H | $CH_3$ | | |
| 147 | 2-Cl,4-F-$C_6H_3$ | $-CH_2-$ | H | $CH_3$ | | |
| 148 | 2-Ethoxy-$C_6H_4$ | $-CH_2-$ | H | $CH_3$ | | |
| 149 | 4-Ethoxy-$C_6H_4$ | $-CH_2-$ | H | $CH_3$ | | |
| 150 | 2-$OCH_3$-$C_6H_4$ | $-CH_2-$ | H | $CH_3$ | | |
| 151 | 4-$OCH_3$-$C_6H_4$ | $-CH_2-$ | H | $CH_3$ | | |
| 152 | 4-t-$C_4H_9$-$C_6H_4$ | $-CH_2-$ | H | $CH_3$ | | |

Tabelle 1 (Fortsetzung)

| Nr. | $R^3$ | $(X)_n$ | $R^2$ | $R^1$ | Fp ($^0C$) | IR ($cm^{-1}$) |
|---|---|---|---|---|---|---|
| 153 | $C_6H_5$ | $-CH(iso-C_3H_7)-$ | H | $CH_3$ | | |
| 154 | $4-Cl-C_6H_4$ | $-CH(iso-C_3H_7)-$ | H | $CH_3$ | | |
| 155 | $4-F-C_6H_4$ | $-CH(iso-C_3H_7)-$ | H | $CH_3$ | | |
| 156 | $4-OCF_2H-C_6H_4$ | $-CH(iso-C_3H_7)-$ | H | $CH_3$ | | |
| 157 | $C_6H_5$ | $-CH(OH)-$ | H | $CH_3$ | | |
| 158 | $2-OCH_3-C_6H_4$ | $-CH(OH)-$ | H | $CH_3$ | | |
| 159 | $3-OCH_3-C_6H_4$ | $-CH(OH)-$ | H | $CH_3$ | | |
| 160 | $4-OCH_3-C_6H_4$ | $-CH(OH)-$ | H | $CH_3$ | | |
| 161 | $4-Cl-C_6H_4$ | $-CH(OH)-$ | H | $CH_3$ | | |
| 162 | $C_6H_5$ | $-CH(CH_2OH)-$ | H | $CH_3$ | | |
| 163 | $C_6H_5$ | $-CH_2-CH_2-$ | H | $CH_3$ | | |
| 164 | $C_6H_5$ | $-CH(CH_3)-CH_2-$ | H | $CH_3$ | | |
| 165 | $C_6H_5$ | $-CH_2-CH(CH_3)-$ | H | $CH_3$ | | |
| 166 | $C_6H_5$ | $-CH(CH_3)-CH(CH_3)-$ | H | $CH_3$ | | |
| 167 | $C_6H_5$ | $-CH(C_6H_5)-CH_2-$ | H | $CH_3$ | | |
| 168 | $4-t-C_4H_9-C_6H_4$ | $-CH_2-CH_2-$ | H | $CH_3$ | | |
| 169 | $4-t-C_4H_9-C_6H_4$ | $-CH_2-CH(CH_3)-$ | H | $CH_3$ | | |
| 170 | $2-Cl-C_6H_4$ | $-CH_2-CH_2-$ | H | $CH_3$ | | |
| 171 | $3-Cl-C_6H_4$ | $-CH_2-CH_2-$ | H | $CH_3$ | | |
| 172 | $4-Cl-C_6H_4$ | $-CH_2-CH_2-$ | H | $CH_3$ | | |
| 173 | $2-F-C_6H_4$ | $-CH_2-CH_2-$ | H | $CH_3$ | | |
| 174 | $3-F-C_6H_4$ | $-CH_2-CH_2-$ | H | $CH_3$ | | |
| 175 | $4-F-C_6H_4$ | $-CH_2-CH_2-$ | H | $CH_3$ | | |
| 176 | $2-OCH_3-C_6H_4$ | $-CH_2-CH_2-$ | H | $CH_3$ | | |
| 177 | $4-OCH_3-C_6H_4$ | $-CH_2-CH_2-$ | H | $CH_3$ | | |
| 178 | $C_6H_5$ | $-CH=CH-$ | H | $CH_3$ | Öl | 1716, 1637; 1254, 1164, 768 |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 179 | $2-Cl-C_6H_4$ | -CH=CH- | H | $CH_3$ | | |
| 180 | $3-Cl-C_6H_4$ | -CH=CH- | H | $CH_3$ | | |
| 181 | $4-Cl-C_6H_4$ | -CH=CH- | H | $CH_3$ | | |
| 182 | $2,6-Cl_2-C_6H_3$ | -CH=CH- | H | $CH_3$ | | |
| 183 | $2,4-Cl_2-C_6H_3$ | -CH=CH- | H | $CH_3$ | | |
| 184 | $2-F-C_6H_4$ | -CH=CH- | H | $CH_3$ | | |
| 185 | $3-F-C_6H_4$ | -CH=CH- | H | $CH_3$ | | |
| 186 | $4-F-C_6H_4$ | -CH=CH- | H | $CH_3$ | | |
| 187 | $2-CF_3-C_6H_4$ | -CH=CH- | H | $CH_3$ | | |
| 188 | $4-CF_3-C_6H_4$ | -CH=CH- | H | $CH_3$ | | |
| 189 | $2-CH_3-C_6H_4$ | -CH=CH- | H | $CH_3$ | | |
| 190 | $4-CH_3-C_8H_4$ | -CH=CH- | H | $CH_3$ | | |
| 191 | $4-i-C_3H_7-C_6H_4$ | -CH=CH- | H | $CH_3$ | | |
| 192 | $4-t-C_4H_9-C_6H_4$ | -CH=CH- | H | $CH_3$ | | |
| 193 | $2-OCH_3-C_6H_4$ | -CH=CH- | H | $CH_3$ | | |
| 194 | $3-OCH_3-C_6H_4$ | -CH=CH- | H | $CH_3$ | | |
| 195 | $4-OCH_3-C_6H_4$ | -CH=CH- | H | $CH_3$ | | |
| 196 | 2-Phenoxy-$C_6H_4$ | -CH=CH- | H | $CH_3$ | | |
| 197 | 3-Phenoxy-$C_6H_4$ | -CH=CH- | H | $CH_3$ | | |
| 198 | 4-Phenoxy-$C_6H_4$ | -CH=CH- | H | $CH_3$ | | |
| 199 | $C_6H_5$ | $-(CH_2)_3-$ | H | $CH_3$ | | |
| 200 | $C_6H_5$ | $-CH(CH_3)-CH_2-CH_2-$ | H | $CH_3$ | | |
| 201 | $C_6H_5$ | $-CH_2-CH(CH_3)-CH_2-$ | H | $CH_3$ | | |
| 202 | $C_6H_5$ | $-CH_2-CH_2-CH(CH_3)-$ | H | $CH_3$ | | |
| 203 | $2-Cl-C_6H_4$ | $-(CH_2)_3-$ | H | $CH_3$ | | |
| 204 | $4-Cl-C_6H_4$ | $-(CH_2)_3-$ | H | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 205 | 2-$OCH_3$-$C_6H_4$ | -$(CH_2)_3$- | H | $CH_3$ | | |
| 206 | 4-$OCH_3$-$C_6H_4$ | -$(CH_2)_3$- | H | $CH_3$ | | |
| 207 | 4-t-$C_4H_9$-$C_6H_4$ | -$(CH_2)_3$- | H | $CH_3$ | | |
| 208 | $C_6H_5$ | -CH=CH-$CH_2$- | H | $CH_3$ | | |
| 209 | $C_6H_5$ | -$(CH_2)_4$- | H | $CH_3$ | | |
| 210 | 2-Cl-$C_6H_4$ | -$(CH_2)_4$- | H | $CH_3$ | | |
| 211 | 4-Cl-$C_6H_4$ | -$(CH_2)_4$- | H | $CH_3$ | | |
| 212 | 2-$OCH_3$-$C_6H_4$ | -$(CH_2)_4$- | H | $CH_3$ | | |
| 213 | 4-$OCH_3$-$C_6H_4$ | -$(CH_2)_4$- | H | $CH_3$ | | |
| 214 | 4-$CF_3$-$C_6H_4$ | -$(CH_2)_4$- | H | $CH_3$ | | |
| 215 | 2-$CH_3$-$C_6H_4$ | -$(CH_2)_4$- | H | $CH_3$ | | |
| 216 | 4-$CH_3$-$C_6H_4$ | -$(CH_2)_4$- | H | $CH_3$ | | |
| 217 | $C_6H_5$ | -$CH_2$-$CH_2$-$CH(CH_3)$-$CH_2$- | H | $CH_3$ | | |
| 218 | $C_6H_5$ | -$(CH_2)_5$- | H | $CH_3$ | | |
| 219 | 2-$CH_3$-$C_6H_4$ | -$(CH_2)_5$- | H | $CH_3$ | | |
| 220 | 4-$CH_3$-$C_6H_4$ | -$(CH_2)_5$- | H | $CH_3$ | | |
| 221 | 2-Cl-$C_6H_4$ | -$(CH_2)_5$- | H | $CH_3$ | | |
| 222 | 4-Cl-$C_6H_4$ | -$(CH_2)_5$- | H | $CH_3$ | | |
| 223 | 2-$OCH_3$-$C_6H_4$ | -$(CH_2)_5$- | H | $CH_3$ | | |
| 224 | 4-$OCH_3$-$C_6H_4$ | -$(CH_2)_5$- | H | $CH_3$ | | |
| 225 | 4-$CF_3$-$C_6H_4$ | -$(CH_2)_5$- | H | $CH_3$ | | |
| 226 | $C_6H_5$ | -$CH_2$-$CH_2$-$CH_2$-$CH(CH_3)$-$CH_2$- | H | $CH_3$ | | |
| 227 | $C_6H_5$ | -$CH_2$-$CH(CH_3)$-$CH_2$-$CH(CH_3)$-$CH_2$- | H | $CH_3$ | | |
| 228 | 4-t-$C_4H_9$-$C_6H_4$ | -$CH_2$-$CH(CH_3)$-$CH_2$-$CH(CH_3)$-$CH_2$- | H | $CH_3$ | | |
| 229 | $C_6H_5$ | -$(CH_2)_6$- | H | $CH_3$ | | |
| 230 | $C_6H_5$ | -$(CH_2)_4$-$CH(CH_3)$-$CH_2$- | H | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 231 | $C_6H_5$-O- | $-CH_2-$ | H | $CH_3$ | | |
| 232 | 2-Cl-$C_6H_4$-O- | $-CH_2-$ | H | $CH_3$ | | |
| 233 | 3-Cl-$C_6H_4$-O- | $-CH_2-$ | H | $CH_3$ | | |
| 234 | 4-Cl-$C_6H_4$-O- | $-CH_2-$ | H | $CH_3$ | | |
| 235 | 2,4-$Cl_2$-$C_6H_3$-O- | $-CH_2-$ | H | $CH_3$ | | |
| 236 | 2-$CH_3$-$C_6H_4$-O- | $-CH_2-$ | H | $CH_3$ | | |
| 237 | 4-$CH_3$-$C_6H_4$-O- | $-CH_2-$ | H | $CH_3$ | | |
| 238 | 2-$OCH_3$-$C_6H_4$-O- | $-CH_2-$ | H | $CH_3$ | | |
| 239 | 4-$OCH_3$-$C_6H_4$-O- | $-CH_2-$ | H | $CH_3$ | | |
| 240 | 4-$CF_3$-$C_6H_4$-O- | $-CH_2-$ | H | $CH_3$ | | |
| 241 | $C_6H_5$-O- | $-CH(CH_3)-$ | H | $CH_3$ | | |
| 242 | $C_6H_5$-O- | $-CH_2-CH_2-$ | H | $CH_3$ | | |
| 243 | 2-Cl-$C_6H_4$-O- | $-CH_2-CH_2-$ | H | $CH_3$ | | |
| 244 | 4-Cl-$C_6H_4$-O- | $-CH_2-CH_2-$ | H | $CH_3$ | | |
| 245 | 2-$CH_3$-$C_6H_4$-O- | $-CH_2-CH_2-$ | H | $CH_3$ | | |
| 246 | 4-$CH_3$-$C_6H_4$-O- | $-CH_2-CH_2-$ | H | $CH_3$ | | |
| 247 | 2-$OCH_3$-$C_6H_4$-O- | $-CH_2-CH_2-$ | H | $CH_3$ | | |
| 248 | 4-$OCH_3$-$C_6H_4$-O- | $-CH_2-CH_2-$ | H | $CH_3$ | | |
| 249 | 4-t-$C_4H_9$-$C_6H_4$-O- | $-CH_2-CH_2-$ | H | $CH_3$ | | |
| 250 | 4-sec.-$C_4H_9$-$C_6H_4$-O- | $-CH_2-CH_2-$ | H | $CH_3$ | | |
| 251 | $C_6H_5$-O- | $-(CH_2)_3-$ | H | $CH_3$ | | |
| 252 | 2-Cl-$C_6H_4$-O- | $-(CH_2)_3-$ | H | $CH_3$ | | |
| 253 | 4-Cl-$C_6H_4$-O- | $-(CH_2)_3-$ | H | $CH_3$ | | |
| 254 | 3-Fl-$C_6H_4$-O- | $-(CH_2)_3-$ | H | $CH_3$ | | |
| 255 | 4-F-$C_6H_4$-O- | $-(CH_2)_3-$ | H | $CH_3$ | | |
| 256 | 2-$CH_3$-$C_6H_4$-O- | $-(CH_2)_3-$ | H | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 257 | $4-CH_3-C_6H_4-O-$ | $-(CH_2)_3-$ | H | $CH_3$ | | |
| 258 | $2-OCH_3-C_6H_4-O-$ | $-(CH_2)_3-$ | H | $CH_3$ | | |
| 259 | $4-OCH_3-C_6H_4-O-$ | $-(CH_2)_3-$ | H | $CH_3$ | | |
| 260 | $2,4-Cl_2-C_6H_3-O-$ | $-(CH_2)_3-$ | H | $CH_3$ | | |
| 261 | $4-Cl-C_6H_4-O-$ | $-CH(CH_3)-CH_2-CH_2-$ | H | $CH_3$ | | |
| 262 | $2-CF_3-C_6H_4-O-$ | $-(CH_2)_3-$ | H | $CH_3$ | | |
| 263 | $3-CF_3-C_6H_4-O-$ | $-(CH_2)_3-$ | H | $CH_3$ | | |
| 264 | $4-CF_3-C_6H_4-O-$ | $-(CH_2)_3-$ | H | $CH_3$ | | |
| 265 | 4-t-Butoxy-$C_6H_4-O-$ | $-(CH_2)_3-$ | H | $CH_3$ | | |
| 266 | $2-CH_3,4-Cl-C_6H_3-O-$ | $-(CH_2)_3-$ | H | $CH_3$ | | |
| 267 | $4-C_2H_5-C_6H_4-O-$ | $-(CH_2)_3-$ | H | $CH_3$ | | |
| 268 | 4-iso-$C_3H_7-C_6H_4-O-$ | $-(CH_2)_3-$ | H | $CH_3$ | | |
| 269 | 4-t-$C_4H_9-C_6H_4-O-$ | $-(CH_2)_3-$ | H | $CH_3$ | | |
| 270 | $C_6H_5-O-$ | $-CH_2-CH(CH_3)-CH_2-$ | H | $CH_3$ | | |
| 271 | $C_6H_5-O-$ | $-(CH_2)_4-$ | H | $CH_3$ | | |
| 272 | $2-Cl-C_6H_4-O-$ | $-(CH_2)_4-$ | H | $CH_3$ | | |
| 273 | $4-Cl-C_6H_4-O-$ | $-(CH_2)_4-$ | H | $CH_3$ | | |
| 274 | $2,4-Cl_2-C_6H_3-O-$ | $-(CH_2)_4-$ | H | $CH_3$ | | |
| 275 | $2,6-Cl_2-C_6H_3-O-$ | $-(CH_2)_4-$ | H | $CH_3$ | | |
| 276 | $2-CH_3-C_6H_4-O-$ | $-(CH_2)_4-$ | H | $CH_3$ | | |
| 277 | $4-CH_3-C_6H_4-O-$ | $-(CH_2)_4-$ | H | $CH_3$ | | |
| 278 | $C_6H_5-O-$ | $-CH_2-CH_2-CH(CH_3)-CH_2-$ | H | $CH_3$ | | |
| 279 | $C_6H_5-O-$ | $-(CH_2)_5-$ | H | $CH_3$ | | |
| 280 | $3-Cl-C_6H_4-O-$ | $-(CH_2)_5-$ | H | $CH_3$ | | |
| 281 | $C_6H_5-O-$ | $-(CH_2)_3-CH(CH_3)-CH_2-$ | H | $CH_3$ | | |
| 282 | $C_6H_5-O-$ | $-(CH_2)_6-$ | H | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 283 | 3-Cl-$C_6H_5$-O- | $-(CH_2)_6-$ | H | $CH_3$ | | |
| 284 | $C_6H_5$-O- | $-(CH_2)_4-CH(CH_3)-CH_2-$ | H | $CH_3$ | | |
| 285 | A 1*) | - | H | $CH_3$ | | |
| 286 | A 2*) | - | H | $CH_3$ | Öl | 2950, 1720, 1434, 1255, 1169, 761 |
| 287 | A 3*) | - | H | $CH_3$ | | |
| 288 | A 4*) | - | H | $CH_3$ | | |
| 289 | A 5*) | - | H | $CH_3$ | | |
| 290 | A 6*) | - | H | $CH_3$ | | |
| 291 | A 7*) | - | H | $CH_3$ | | |
| 292 | A 8*) | - | H | $CH_3$ | | |
| 293 | A 9*) | - | H | $CH_3$ | | |
| 294 | A 10*) | - | H | $CH_3$ | | |
| 295 | A 11*) | - | H | $CH_3$ | | |
| 296 | A 12*) | - | H | $CH_3$ | | |
| 297 | A 13*) | - | H | $CH_3$ | | |
| 298 | A 14*) | - | H | $CH_3$ | | |
| 299 | A 15*) | - | H | $CH_3$ | | |
| 300 | A 15*) | - | H | $CH_3$ | | |
| 301 | A 17*) | - | H | $CH_3$ | | |
| 302 | N-Pyrrolyl | $-CH(iso-C_3H_7)-$ | H | $CH_3$ | | |
| 303 | 4-tert.-Butyl-$C_6H_4$ | $-CH_2-C(CH_3)=CH-CH=CH-$ | H | $CH_3$ | | |
| 304 | H | $-CH_2-CH(CH_3)-CH_2-CH(CH_3)-$ | H | $CH_3$ | | |
| 305 | H | $-CH_2-CH(CH_3)-CH_2-CH(C_2H_5)-$ | H | $CH_3$ | | |
| 306 | H | $-CH_2-CH(CH_3)-CH_2-CH(n-C_3H_7)-$ | H | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 307 | H | $-CH_2-CH(CH_3)-CH_2-CH(i-C_3H_7)-$ | H | $CH_3$ | | |
| 308 | H | $-CH_2-C(CH_3)_2-CH_2-CH(CH_3)-CH_2-$ | H | $CH_3$ | | |
| 309 | H | $-(CH_2)_5-CH(C_2H_5)-$ | H | $CH_3$ | | |
| 310 | H | $-(CH_2)_5-CH(n-C_3H_7)-$ | H | $CH_3$ | | |
| 311 | H | $-(CH_2)_4-O-CH_2-C(CH_3)_2-$ | H | $CH_3$ | | |
| 312 | H | $-CH_2-O-CH_2-C(CH_3)_2-$ | H | $CH_3$ | | |
| 313 | $C_6H_5$ | $-CH=CH-(CH_2)_4-$ | H | $CH_3$ | | |
| 314 | H | $-CH_2-C(CH_3)_2-CH_2-CH(CH_3)_2-CH_2-$ | H | $CH_3$ | | |
| 315 | H | $-CH_2-CH(CH_3)-(CH_2)_2-CH(i-C_3H_7)-$ | H | $CH_3$ | | |
| 316 | 1-Methylcyclopropyl | - | H | $CH_3$ | Öl | 2955,1720,1322, 1255,1156,1038, 758 |
| 317 | 2-Methylcyclopropyl | - | H | $CH_3$ | | |
| 318 | 2-Phenylcyclopropyl | - | H | $CH_3$ | | |
| 319 | 1-Methylcyclohexyl | - | H | $CH_3$ | | |
| 320 | $2-Cl-C_6H_4$ | -CHCl- | H | $CH_3$ | | |
| 321 | 1-Methylcyclopropyl | - | H | $C_2H_5$ | | |
| 322 | 1-Methylcyclopropyl | - | H | $i-C_3H_7$ | | |
| 323 | 1-Methylcyclopropyl | - | $C_2H_5$ | $CH_3$ | | |
| 324 | 1-Methylcyclopropyl | - | $C_3H_7$ | $CH_3$ | | |
| 325 | 1-Methylcyclopropyl | - | $C_4H_9$ | $CH_3$ | | |
| 326 | 1-Methylcyclopropyl | - | $OCH_3$ | $CH_3$ | | |
| 327 | 1-Methylcyclopropyl | - | $OC_2H_5$ | $CH_3$ | | |
| 328 | 1-Methylcyclopropyl | - | $O-i-C_3H_7$ | $CH_3$ | | |
| 329 | 1-Methylcyclopropyl | - | $OC_4H_9$ | $CH_3$ | | |
| 330 | H | $-(CH_2)_4-CH(CH_3)-CH_2-$ | $OCH_3$ | $CH_3$ | | |
| 331 | H | $-C(CH_3)_2-$ | $OCH_3$ | $CH_3$ | | |
| 332 | $C_6H_5$ | - | $OCH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 333 | $C_6H_5$ | $-CH(CH_3)-$ | $OCH_3$ | $CH_3$ | | |
| 334 | 1-Methylcyclohexyl | - | $OCH_3$ | $CH_3$ | | |
| 335 | 1-Ethylcyclohexyl | - | H | $CH_3$ | | |
| 336 | H | $-CH_2-$ | $CH_3$ | $CH_3$ | | |
| 337 | H | $-CH_2-CH_2-$ | $CH_3$ | $CH_3$ | | |
| 338 | H | $-CH_2-CH(CH_3)-$ | $CH_3$ | $CH_3$ | | |
| 339 | H | $-CH_2-C(CH_3)_2-$ | $CH_3$ | $CH_3$ | Öl | 1722, 1281, 1243, 1176 |
| 340 | H | $-CH=CH-$ | $CH_3$ | $CH_3$ | | |
| 341 | H | $-CH=C(CH_3)-$ | $CH_3$ | $CH_3$ | | |
| 342 | H | $-C\equiv C-$ | $CH_3$ | $CH_3$ | | |
| 343 | H | $-CH_2-CH_2-CH_2-$ | $CH_3$ | $CH_3$ | | |
| 344 | H | $-CH_2-CH_2-CH(CH_3)-$ | $CH_3$ | $CH_3$ | | |
| 345 | H | $-CH_2-CH(CH_3)-CH_2-$ | $CH_3$ | $CH_3$ | | |
| 346 | H | $-CH_2-CH_2-C(CH_3)_2-$ | $CH_3$ | $CH_3$ | | |
| 347 | H | $-CH_2-C(CH_3)_2-CH_2-$ | $CH_3$ | $CH_3$ | | |
| 348 | H | $-CH_2-CH_2-C(C_2H_5)_2$ | $CH_3$ | $CH_3$ | | |
| 349 | H | $-CH=CH-CH_2-$ | $CH_3$ | $CH_3$ | | |
| 350 | H | $-CH_2-CH=CH-$ | $CH_3$ | $CH_3$ | | |
| 351 | H | $-CH_2-C(CH_3)=CH-$ | $CH_3$ | $CH_3$ | | |
| 352 | H | $-CH_2-CH=C(CH_3)-$ | $CH_3$ | $CH_3$ | | |
| 353 | H | $-(CH_2)_4-$ | $CH_3$ | $CH_3$ | | |
| 354 | H | $-CH_2-CH_2-CH_2-CH(CH_3)-$ | $CH_3$ | $CH_3$ | | |
| 355 | H | $-CH_2-CH(CH_3)-CH_2-CH_2-$ | $CH_3$ | $CH_3$ | | |
| 356 | H | $-(CH_2)_3-C(CH_3)_2-$ | $CH_3$ | $CH_3$ | | |
| 357 | H | $-(CH_2)_3-CH(C_2H_5)-$ | $CH_3$ | $CH_3$ | | |
| 358 | H | $-(CH_2)_3-CH(n-C_3H_7)-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 359 | H | $-CH_2-CH=CH-CH_2-$ | $CH_3$ | $CH_3$ | | |
| 360 | H | $-CH_2-CH(CH_3)=CH-CH_2-$ | $CH_3$ | $CH_3$ | | |
| 361 | H | $-(CH_2)_5-$ | $CH_3$ | $CH_3$ | | |
| 362 | H | $-(CH_2)_4-CH(CH_3)-$ | $CH_3$ | $CH_3$ | | |
| 363 | H | $-(CH_2)_4-CH(C_2H_5)-$ | $CH_3$ | $CH_3$ | | |
| 364 | H | $-(CH_2)_3-CH(CH_3)-CH_2-$ | $CH_3$ | $CH_3$ | | |
| 365 | H | $-CH_2-CH=CH-CH=CH-$ | $CH_3$ | $CH_3$ | | |
| 366 | H | $-CH_2-C(CH_3)=CH-CH=CH-$ | $CH_3$ | $CH_3$ | | |
| 367 | H | $-(CH_2)_6-$ | $CH_3$ | $CH_3$ | | |
| 368 | H | $-(CH_2)_5-CH(CH_3)-$ | $CH_3$ | $CH_3$ | | |
| 369 | H | $-(CH_2)_4-CH(CH_3)-CH_2-$ | $CH_3$ | $CH_3$ | | |
| 370 | H | $-(CH_2)_5-CH(n-C_3H_7)-$ | $CH_3$ | $CH_3$ | | |
| 371 | H | $-(CH_2)_7-$ | $CH_3$ | $CH_3$ | | |
| 372 | H | $-(CH_2)_6-CH(CH_3)-$ | $CH_3$ | $CH_3$ | | |
| 373 | H | $-(CH_2)_5-CH(CH_3)-CH_2-$ | $CH_3$ | $CH_3$ | | |
| 374 | H | $-(CH_2)_6-C(CH_3)_2-$ | $CH_3$ | $CH_3$ | | |
| 375 | H | $-(CH_2)_8-$ | $CH_3$ | $CH_3$ | | |
| 376 | H | $-(CH_2)_9-$ | $CH_3$ | $CH_3$ | | |
| 377 | H | $-(CH_2)_{10}-$ | $CH_3$ | $CH_3$ | | |
| 378 | H | $-CHCl-$ | $CH_3$ | $CH_3$ | | |
| 379 | H | $-CCl_2-$ | $CH_3$ | $CH_3$ | | |
| 380 | Cl | $-CCl_2-$ | $CH_3$ | $CH_3$ | | |
| 381 | H | $-CHBr-$ | $CH_3$ | $CH_3$ | | |
| 382 | H | $-CBr_2-$ | $CH_3$ | $CH_3$ | | |
| 383 | Br | $-CBr_2$ | $CH_3$ | $CH_3$ | | |
| 384 | H | $-CHF-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 385 | H | $-CF_2-$ | $CH_3$ | $CH_3$ | | |
| 386 | F | $-CF_2-$ | $CH_3$ | $CH_3$ | | |
| 387 | H | $-CH=CCl-$ | $CH_3$ | $CH_3$ | | |
| 388 | H | $-CCl=CCl-$ | $CH_3$ | $CH_3$ | | |
| 389 | Cl | $-C(CH_3)_2-$ | $CH_3$ | $CH_3$ | | |
| 390 | H | $-C(CH_3)_2-$ | $CH_3$ | $CH_3$ | | |
| 391 | H | $-CHCl-CH(CH_3)-$ | $CH_3$ | $CH_3$ | | |
| 392 | H | $-CHCl-C(CH_3)_2-$ | $CH_3$ | $CH_3$ | | |
| 393 | H | $-CHBr-CH(CH_3)-$ | $CH_3$ | $CH_3$ | | |
| 394 | Br | $-C(C_2H_5)_2-$ | $CH_3$ | $CH_3$ | | |
| 395 | H | $-CH(OH)-$ | $CH_3$ | $CH_3$ | | |
| 396 | H | $-CH_2-CH(OH)-$ | $CH_3$ | $CH_3$ | | |
| 397 | H | $-CH_2-CH_2-CH(OH)-$ | $CH_3$ | $CH_3$ | | |
| 398 | H | $-CH_2-CH(OH)-CH_2-$ | $CH_3$ | $CH_3$ | | |
| 399 | H | $-CH(OH)-CH_2-$ | $CH_3$ | $CH_3$ | | |
| 400 | H | $-CH(OH)-C(CH_3)_2-$ | $CH_3$ | $CH_3$ | | |
| 401 | H | $-CH_2-C(OH)(CH_3)-$ | $CH_3$ | $CH_3$ | | |
| 402 | H | $-CH_2-CH(CH_3)-CH(OH)-$ | $CH_3$ | $CH_3$ | | |
| 403 | H | $-CH=CH-CH(OH)-$ | $CH_3$ | $CH_3$ | | |
| 404 | H | $-CH=CH-CH_2-CH(OH)-$ | $CH_3$ | $CH_3$ | | |
| 405 | CN | $-CH_2-$ | $CH_3$ | $CH_3$ | | |
| 406 | Cyclopropyl | - | $CH_3$ | $CH_3$ | Öl | 1720, 1398, 1244, 1169 |
| 407 | Cyclobutyl | - | $CH_3$ | $CH_3$ | | |
| 408 | Cyclopentyl | - | $CH_3$ | $CH_3$ | | |
| 409 | Cyclohexyl | - | $CH_3$ | $CH_3$ | | |
| 410 | Adamantyl | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 411 | 9-Fluorenyl | - | $CH_3$ | $CH_3$ | | |
| 412 | Cyclopentyl | $-CH_2-$ | $CH_3$ | $CH_3$ | | |
| 413 | 3-Cyclopentenyl | $-CH_2-$ | $CH_3$ | $CH_3$ | | |
| 414 | Cyclohexyl | $-CH_2-$ | $CH_3$ | $CH_3$ | | |
| 415 | Cyclopentyl | $-CH_2-CH_2-$ | $CH_3$ | $CH_3$ | | |
| 416 | Cyclohexyl | $-CH_2-CH_2-$ | $CH_3$ | $CH_3$ | | |
| 417 | Cyclohexyl | $-(CH_2)_3-$ | $CH_3$ | $CH_3$ | | |
| 418 | $C_6H_5$ (=Phenyl) | - | $CH_3$ | $CH_3$ | | |
| 419 | $2-CH_3-C_6H_4$ | - | $CH_3$ | $CH_3$ | | |
| 420 | $3-CH_3-C_6H_4$ | - | $CH_3$ | $CH_3$ | | |
| 421 | $4-CH_3-C_6H_4$ | - | $CH_3$ | $CH_3$ | | |
| 422 | $2,3-(CH_3)_2-C_6H_3$ | - | $CH_3$ | $CH_3$ | | |
| 423 | $2,4-(CH_3)_2-C_6H_3$ | - | $CH_3$ | $CH_3$ | | |
| 424 | $2,6-(CH_3)_2-C_6H_3$ | - | $CH_3$ | $CH_3$ | | |
| 425 | $3,4-(CH_3)_2-C_6H_3$ | - | $CH_3$ | $CH_3$ | | |
| 426 | $3,5-(CH_3)_2-C_6H_3$ | - | $CH_3$ | $CH_3$ | | |
| 427 | $2,4,6-(CH_3)_2-C_6H_2$ | - | $CH_3$ | $CH_3$ | | |
| 428 | $4-t-C_4H_9-C_6H_4$ | - | $CH_3$ | $CH_3$ | | |
| 429 | $2-C_6H_5-C_6H_4$ | - | $CH_3$ | $CH_3$ | | |
| 430 | $4-C_6H_5-C_6H_4$ | - | $CH_3$ | $CH_3$ | | |
| 431 | 2-Benzyl-$C_6H_4$ | - | $CH_3$ | $CH_3$ | | |
| 432 | 4-Benzyl-$C_6H_4$ | - | $CH_3$ | $CH_3$ | | |
| 433 | $2-Cl-C_6H_4$ | - | $CH_3$ | $CH_3$ | | |
| 434 | $3-Cl-C_6H_4$ | - | $CH_3$ | $CH_3$ | | |
| 435 | $4-Cl-C_6H_4$ | - | $CH_3$ | $CH_3$ | | |
| 436 | $2,4-Cl_2-C_6H_3$ | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 437 | $2,5-Cl_2-C_6H_3$ | - | $CH_3$ | $CH_3$ | | |
| 438 | $2,6-Cl_2-C_6H_3$ | - | $CH_3$ | $CH_3$ | | |
| 439 | $3,4-Cl_2-C_6H_3$ | - | $CH_3$ | $CH_3$ | | |
| 440 | $3,5-Cl_2-C_6H_3$ | - | $CH_3$ | $CH_3$ | | |
| 441 | $2,4,5-Cl3-C_6H_2$ | - | $CH_3$ | $CH_3$ | | |
| 442 | $2,3,4,5,6-Cl_5-C_6$ | - | $CH_3$ | $CH_3$ | | |
| 443 | $2-F,4-Cl-C_6H_3$ | - | $CH_3$ | $CH_3$ | | |
| 444 | $2-F-C_6H_4$ | - | $CH_3$ | $CH_3$ | | |
| 445 | $3-F-C_6H_4$ | - | $CH_3$ | $CH_3$ | | |
| 446 | $4-F-C_6H_4$ | - | $CH_3$ | $CH_3$ | | |
| 447 | $2,4-F_2-C_6H_3$ | - | $CH_3$ | $CH_3$ | | |
| 448 | $2,6-F_2-C_6H_3$ | - | $CH_3$ | $CH_3$ | | |
| 449 | $2,3,4,5,6-F_5-C_6$ | - | $CH_3$ | $CH_3$ | | |
| 450 | $2-CF_3-C_6H_4$ | - | $CH_3$ | $CH_3$ | | |
| 451 | $3-CF_3-C_6H_4$ | - | $CH_3$ | $CH_3$ | | |
| 452 | $4-CF_3-C_6H_4$ | - | $CH_3$ | $CH_3$ | | |
| 453 | $2-OCH_3-C_6H_4$ | - | $CH_3$ | $CH_3$ | | |
| 454 | $3-OCH_3-C_6H_4$ | - | $CH_3$ | $CH_3$ | | |
| 455 | $4-OCH_3-C_6H_4$ | - | $CH_3$ | $CH_3$ | | |
| 456 | 2-Phenoxy-$C_6H_4$ | - | $CH_3$ | $CH_3$ | | |
| 457 | 3-Phenoxy-$C_6H_4$ | - | $CH_3$ | $CH_3$ | | |
| 458 | 4-Phenoxy-$C_6H_4$ | - | $CH_3$ | $CH_3$ | | |
| 459 | 4-Ethoxy-$C_6H_4$ | - | $CH_3$ | $CH_3$ | | |
| 460 | 2-Phenoxyethoxy-$C_6H_4$ | - | $CH_3$ | $CH_3$ | | |
| 461 | 2-(2'-Cl-Phenoxyethoxy)-$C_6H_4$ | - | $CH_3$ | $CH_3$ | | |
| 462 | 2-(3'-Cl-Phenoxyethoxy)-$C_6H_4$ | - | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 463 | 2-(4'-Cl-Phenoxyethoxy)-$C_6H_4$ | - | $CH_3$ | $CH_3$ | | |
| 464 | 3-Phenoxyethoxy-$C_6H_4$ | - | $CH_3$ | $CH_3$ | | |
| 465 | 3-(4'-Cl-Phenoxyethoxy)-$C_6H_4$ | - | $CH_3$ | $CH_3$ | | |
| 466 | 4-Phenoxyethoxy-$C_6H_4$ | - | $CH_3$ | $CH_3$ | | |
| 467 | 2-Phenoxypropoxy-$C_6H_4$ | - | $CH_3$ | $CH_3$ | | |
| 468 | 3-Phenoxypropoxy-$C_6H_4$ | - | $CH_3$ | $CH_3$ | | |
| 469 | 4-Phenoxypropoxy-$C_6H_4$ | - | $CH_3$ | $CH_3$ | | |
| 470 | $C_6H_5$ | $-CH_2-$ | $CH_3$ | $CH_3$ | | |
| 471 | 2-$CH_3$-$C_6H_4$ | $-CH_2-$ | $CH_3$ | $CH_3$ | | |
| 472 | $C_6H_5$ | $-CH(CH_3)-$ | $CH_3$ | $CH_3$ | | |
| 473 | 4-Phenyl-$C_6H_4$ | $-CH_2-$ | $CH_3$ | $CH_3$ | | |
| 474 | 2-F-$C_6H_4$ | $-CH_2-$ | $CH_3$ | $CH_3$ | | |
| 475 | 3-F-$C_6H_4$ | $-CH_2-$ | $CH_3$ | $CH_3$ | | |
| 476 | 4-F-$C_6H_4$ | $-CH_2-$ | $CH_3$ | $CH_3$ | | |
| 477 | 2-Cl-$C_6H_4$ | $-CH_2-$ | $CH_3$ | $CH_3$ | | |
| 478 | 3-Cl-$C_6H_4$ | $-CH_2-$ | $CH_3$ | $CH_3$ | | |
| 479 | 4-Cl-$C_6H_4$ | $-CH_2-$ | $CH_3$ | $CH_3$ | | |
| 480 | 2,4-$Cl_2$-$C_6H_3$ | $-CH_2-$ | $CH_3$ | $CH_3$ | | |
| 481 | 2,6-$Cl_2$-$C_6H_3$ | $-CH_2-$ | $CH_3$ | $CH_3$ | | |
| 482 | 2-Cl,4-F-$C_6H_3$ | $-CH_2-$ | $CH_3$ | $CH_3$ | | |
| 483 | 2-Ethoxy-$C_6H_4$ | $-CH_2-$ | $CH_3$ | $CH_3$ | | |
| 484 | 4-Ethoxy-$C_6H_4$ | $-CH_2-$ | $CH_3$ | $CH_3$ | | |
| 485 | 2-$OCH_3$-$C_6H_4$ | $-CH_2-$ | $CH_3$ | $CH_3$ | | |
| 486 | 4-$OCH_3$-$C_6H_4$ | $-CH_2-$ | $CH_3$ | $CH_3$ | | |
| 487 | 4-t-$C_4H_9$-$C_6H_4$ | $-CH_2-$ | $CH_3$ | $CH_3$ | | |
| 488 | $C_6H_5$ | -CH(iso-$C_3H_7$)- | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 489 | $4-Cl-C_6H_4$ | $-CH(iso-C_3H_7)-$ | $CH_3$ | $CH_3$ | | |
| 490 | $4-F-C_6H_4$ | $-CH(iso-C_3H_7)-$ | $CH_3$ | $CH_3$ | | |
| 491 | $4-OCF_2H-C_6H_4$ | $-CH(iso-C_3H_7)-$ | $CH_3$ | $CH_3$ | | |
| 492 | $C_6H_5$ | $-CH(OH)-$ | $CH_3$ | $CH_3$ | | |
| 493 | $2-OCH_3-C_6H_4$ | $-CH(OH)-$ | $CH_3$ | $CH_3$ | | |
| 494 | $3-OCH_3-C_6H_4$ | $-CH(OH)-$ | $CH_3$ | $CH_3$ | | |
| 495 | $4-OCH_3-C_6H_4$ | $-CH(OH)-$ | $CH_3$ | $CH_3$ | | |
| 496 | $4-Cl-C_6H_4$ | $-CH(OH)-$ | $CH_3$ | $CH_3$ | | |
| 497 | $C_6H_5$ | $-CH(CH_2OH)-$ | $CH_3$ | $CH_3$ | | |
| 498 | $C_6H_5$ | $-CH_2-CH_2-$ | $CH_3$ | $CH_3$ | | |
| 499 | $C_6H_5$ | $-CH(CH_3)-CH_2-$ | $CH_3$ | $CH_3$ | | |
| 500 | $C_6H_5$ | $-CH_2-CH(CH_3)-$ | $CH_3$ | $CH_3$ | | |
| 501 | $C_6H_5$ | $-CH(CH_3)-CH(CH_3)-$ | $CH_3$ | $CH_3$ | | |
| 502 | $C_6H_5$ | $-CH(C_6H_5)-CH_2-$ | $CH_3$ | $CH_3$ | | |
| 503 | $4-t-C_4H_9-C_6H_4$ | $-CH_2-CH_2-$ | $CH_3$ | $CH_3$ | | |
| 504 | $4-t-C_4H_9-C_6H_4$ | $-CH_2-CH(CH_3)-$ | $CH_3$ | $CH_3$ | | |
| 505 | $2-Cl-C_6H_4$ | $-CH_2-CH_2-$ | $CH_3$ | $CH_3$ | | |
| 506 | $3-Cl-C_6H_4$ | $-CH_2-CH_2-$ | $CH_3$ | $CH_3$ | | |
| 507 | $4-Cl-C_6H_4$ | $-CH_2-CH_2-$ | $CH_3$ | $CH_3$ | | |
| 508 | $2-F-C_6H_4$ | $-CH_2-CH_2-$ | $CH_3$ | $CH_3$ | | |
| 509 | $3-F-C_6H_4$ | $-CH_2-CH_2-$ | $CH_3$ | $CH_3$ | | |
| 510 | $4-F-C_6H_4$ | $-CH_2-CH_2-$ | $CH_3$ | $CH_3$ | | |
| 511 | $2-OCH_3-C_6H_4$ | $-CH_2-CH_2-$ | $CH_3$ | $CH_3$ | | |
| 512 | $4-OCH_3-C_6H_4$ | $-CH_2-CH_2-$ | $CH_3$ | $CH_3$ | | |
| 513 | $C_6H_5$ | $-CH=CH-$ | $CH_3$ | $CH_3$ | Öl | 1716, 1637, 1244, 1164, 767 |
| 514 | $2-Cl-C_6H_4$ | $-CH=CH-$ | $CH_3$ | $CH_3$ | | |

Tabelle 1 (Fortsetzung)

| Nr. | $R^3$ | $(X)_n$ | $R^2$ | $R^1$ | Fp ($^0C$) | IR ($cm^{-1}$) |
|---|---|---|---|---|---|---|
| 515 | $3-Cl-C_6H_4$ | $-CH=CH-$ | $CH_3$ | $CH_3$ | | |
| 516 | $4-Cl-C_6H_4$ | $-CH=CH-$ | $CH_3$ | $CH_3$ | | |
| 517 | $2,6-Cl_2-C_6H_3$ | $-CH=CH-$ | $CH_3$ | $CH_3$ | | |
| 518 | $2,4-Cl_2-C_6H_3$ | $-CH=CH-$ | $CH_3$ | $CH_3$ | | |
| 519 | $2-F-C_6H_4$ | $-CH=CH-$ | $CH_3$ | $CH_3$ | | |
| 520 | $3-F-C_6H_4$ | $-CH=CH-$ | $CH_3$ | $CH_3$ | | |
| 521 | $4-F-C_6H_4$ | $-CH=CH-$ | $CH_3$ | $CH_3$ | | |
| 522 | $2-CF_3-C_6H_4$ | $-CH=CH-$ | $CH_3$ | $CH_3$ | | |
| 523 | $4-CF_3-C_6H_4$ | $-CH=CH-$ | $CH_3$ | $CH_3$ | | |
| 524 | $2-CH_3-C_6H_4$ | $-CH=CH-$ | $CH_3$ | $CH_3$ | | |
| 525 | $4-CH_3-C_6H_4$ | $-CH=CH-$ | $CH_3$ | $CH_3$ | | |
| 526 | $4-i-C_3H_7-C_6H_4$ | $-CH=CH-$ | $CH_3$ | $CH_3$ | | |
| 527 | $4-t-C_4H_9-C_6H_4$ | $-CH=CH-$ | $CH_3$ | $CH_3$ | | |
| 528 | $2-OCH_3-C_6H_4$ | $-CH=CH-$ | $CH_3$ | $CH_3$ | | |
| 529 | $3-OCH_3-C_6H_4$ | $-CH=CH-$ | $CH_3$ | $CH_3$ | | |
| 530 | $4-OCH_3-C_6H_4$ | $-CH=CH-$ | $CH_3$ | $CH_3$ | | |
| 351 | $2-Phenoxy-C_6H_4$ | $-CH=CH-$ | $CH_3$ | $CH_3$ | | |
| 532 | $3-Phenoxy-C_6H_4$ | $-CH=CH-$ | $CH_3$ | $CH_3$ | | |
| 533 | $4-Phenoxy-C_6H_4$ | $-CH=CH-$ | $CH_3$ | $CH_3$ | | |
| 534 | $C_6H_5$ | $-(CH_2)_3-$ | $CH_3$ | $CH_3$ | | |
| 535 | $C_6H_5$ | $-CH(CH_3)-CH_2-CH_2-$ | $CH_3$ | $CH_3$ | | |
| 536 | $C_6H_5$ | $-CH_2-CH(CH_3)-CH_2-$ | $CH_3$ | $CH_3$ | | |
| 537 | $C_6H_5$ | $-CH_2-CH_2-CH(CH_3)-$ | $CH_3$ | $CH_3$ | | |
| 538 | $2-Cl-C_6H_4$ | $-(CH_2)_3-$ | $CH_3$ | $CH_3$ | | |
| 539 | $4-Cl-C_6H_4$ | $-(CH_2)_3-$ | $CH_3$ | $CH_3$ | | |
| 540 | $2-OCH_3-C_6H_4$ | $-(CH_2)_3-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 541 | $4-OCH_3-C_6H_4$ | $-(CH_2)_3-$ | $CH_3$ | $CH_3$ | | |
| 542 | $4-t-C_4H_9-C_6H_4$ | $-(CH_2)_3-$ | $CH_3$ | $CH_3$ | | |
| 543 | $C_6H_5$ | $-CH=CH-CH_2-$ | $CH_3$ | $CH_3$ | | |
| 544 | $C_6H_5$ | $-(CH_2)_4-$ | $CH_3$ | $CH_3$ | | |
| 545 | $2-Cl-C_6H_4$ | $-(CH_2)_4-$ | $CH_3$ | $CH_3$ | | |
| 546 | $4-Cl-C_6H_4$ | $-(CH_2)_4-$ | $CH_3$ | $CH_3$ | | |
| 547 | $2-OCH_3-C_6H_4$ | $-(CH_2)_4-$ | $CH_3$ | $CH_3$ | | |
| 548 | $4-OCH_3-C_6H_4$ | $-(CH_2)_4-$ | $CH_3$ | $CH_3$ | | |
| 549 | $4-CF_3-C_6H_4$ | $-(CH_2)_4-$ | $CH_3$ | $CH_3$ | | |
| 550 | $2-CH_3-C_6H_4$ | $-(CH_2)_4-$ | $CH_3$ | $CH_3$ | | |
| 551 | $4-CH_3-C_6H_4$ | $-(CH_2)_4-$ | $CH_3$ | $CH_3$ | | |
| 552 | $C_6H_5$ | $-CH_2-CH_2-CH(CH_3)-CH_2-$ | $CH_3$ | $CH_3$ | | |
| 553 | $C_6H_5$ | $-(CH_2)_5-$ | $CH_3$ | $CH_3$ | | |
| 554 | $2-CH_3-C_6H_4$ | $-(CH_2)_5-$ | $CH_3$ | $CH_3$ | | |
| 555 | $4-CH_3-C_6H_4$ | $-(CH_2)_5-$ | $CH_3$ | $CH_3$ | | |
| 556 | $2-Cl-C_6H_4$ | $-(CH_2)_5-$ | $CH_3$ | $CH_3$ | | |
| 557 | $4-Cl-C_6H_4$ | $-(CH_2)_5-$ | $CH_3$ | $CH_3$ | | |
| 558 | $2-OCH_3-C_6H_4$ | $-(CH_2)_5-$ | $CH_3$ | $CH_3$ | | |
| 559 | $4-OCH_3-C_6H_4$ | $-(CH_2)_5-$ | $CH_3$ | $CH_3$ | | |
| 560 | $4-CF_3-C_6H_4$ | $-(CH_2)_5-$ | $CH_3$ | $CH_3$ | | |
| 561 | $C_6H_5$ | $-CH_2-CH_2-CH_2-CH(CH_3)-CH_2-$ | $CH_3$ | $CH_3$ | | |
| 562 | $C_6H_5$ | $-CH_2-CH(CH_3)-CH_2-CH(CH_3)-CH_2$ | $CH_3$ | $CH_3$ | | |
| 563 | $4-t-C_4H_9-C_6H_4$ | $-CH_2-CH(CH_3)-CH_2-CH(CH_3)-CH_2-$ | $CH_3$ | $CH_3$ | | |
| 564 | $C_6H_5$ | $-(CH_2)_6-$ | $CH_3$ | $CH_3$ | | |
| 565 | $C_6H_5$ | $-(CH_2)_4-CH(CH_3)-CH_2-$ | $CH_3$ | $CH_3$ | | |
| 566 | $C_6H_5-O-$ | $-CH_2-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 567 | $2-Cl-C_6H_4-O-$ | $-CH_2-$ | $CH_3$ | $CH_3$ | | |
| 568 | $3-Cl-C_6H_4-O-$ | $-CH_2-$ | $CH_3$ | $CH_3$ | | |
| 569 | $4-Cl-C_6H_4-O-$ | $-CH_2-$ | $CH_3$ | $CH_3$ | | |
| 570 | $2,4-Cl_2-C_6H_3-O-$ | $-CH_2-$ | $CH_3$ | $CH_3$ | | |
| 571 | $2-CH_3-C_6H_4-O-$ | $-CH_2-$ | $CH_3$ | $CH_3$ | | |
| 572 | $4-CH_3-C_6H_4-O-$ | $-CH_2-$ | $CH_3$ | $CH_3$ | | |
| 573 | $2-OCH_3-C_6H_4-O-$ | $-CH_2-$ | $CH_3$ | $CH_3$ | | |
| 574 | $4-OCH_3-C_6H_4-O-$ | $-CH_2-$ | $CH_3$ | $CH_3$ | | |
| 575 | $4-CF_3-C_6H_4-O-$ | $-CH_2-$ | $CH_3$ | $CH_3$ | | |
| 576 | $C_6H_5-O-$ | $-CH(CH_3)-$ | $CH_3$ | $CH_3$ | | |
| 577 | $C_6H_5-O-$ | $-CH_2-CH_2-$ | $CH_3$ | $CH_3$ | | |
| 578 | $2-Cl-C_6H_4-O-$ | $-CH_2-CH_2-$ | $CH_3$ | $CH_3$ | | |
| 579 | $4-Cl-C_6H_4-O-$ | $-CH_2-CH_2-$ | $CH_3$ | $CH_3$ | | |
| 580 | $2-CH_3-C_6H_4-O-$ | $-CH_2-CH_2-$ | $CH_3$ | $CH_3$ | | |
| 581 | $4-CH_3-C_6H_4-O-$ | $-CH_2-CH_2-$ | $CH_3$ | $CH_3$ | | |
| 582 | $2-OCH_3-C_6H_4-O-$ | $-CH_2-CH_2-$ | $CH_3$ | $CH_3$ | | |
| 583 | $4-OCH_3-C_6H_4-O-$ | $-CH_2-CH_2-$ | $CH_3$ | $CH_3$ | | |
| 584 | $4-t-C_4H_9-C_6H_4-O-$ | $-CH_2-CH_2-$ | $CH_3$ | $CH_3$ | | |
| 585 | $4-sec.-C_4H_9-C_6H_4-O-$ | $-CH_2-CH_2-$ | $CH_3$ | $CH_3$ | | |
| 586 | $C_6H_5-O-$ | $-(CH_2)_3-$ | $CH_3$ | $CH_3$ | | |
| 587 | $2-Cl-C_6H_4-O-$ | $-(CH_2)_3-$ | $CH_3$ | $CH_3$ | | |
| 588 | $4-Cl-C_6H_4-O-$ | $-(CH_2)_3-$ | $CH_3$ | $CH_3$ | | |
| 589 | $3-F-C_6H_4-O-$ | $-(CH_2)_3-$ | $CH_3$ | $CH_3$ | | |
| 590 | $4-F-C_6H_4-O-$ | $-(CH_2)_3-$ | $CH_3$ | $CH_3$ | | |
| 591 | $2-CH_3-C_6H_4-O-$ | $-(CH_2)_3-$ | $CH_3$ | $CH_3$ | | |
| 592 | $4-CH_3-C_6H_4-O-$ | $-(CH_2)_3-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 593 | $2\text{-}OCH_3\text{-}C_6H_4\text{-}O\text{-}$ | $-(CH_2)_3-$ | $CH_3$ | $CH_3$ | | |
| 594 | $4\text{-}OCH_3\text{-}C_6H_4\text{-}O\text{-}$ | $-(CH_2)_3-$ | $CH_3$ | $CH_3$ | | |
| 595 | $2,4\text{-}Cl_2\text{-}C_6H_3\text{-}O\text{-}$ | $-(CH_2)_3-$ | $CH_3$ | $CH_3$ | | |
| 596 | $4\text{-}Cl\text{-}C_6H_4\text{-}O\text{-}$ | $-CH(CH_3)-CH_2-CH_2-$ | $CH_3$ | $CH_3$ | | |
| 597 | $2\text{-}CF_3\text{-}C_6H_4\text{-}O\text{-}$ | $-(CH_2)_3-$ | $CH_3$ | $CH_3$ | | |
| 598 | $3\text{-}CF_3\text{-}C_6H_4\text{-}O\text{-}$ | $-(CH_2)_3-$ | $CH_3$ | $CH_3$ | | |
| 599 | $4\text{-}CF_3\text{-}C_6H_4\text{-}O\text{-}$ | $-(CH_2)_3-$ | $CH_3$ | $CH_3$ | | |
| 600 | $4\text{-}t\text{-}Butoxy\text{-}C_6H_4\text{-}O\text{-}$ | $-(CH_2)_3-$ | $CH_3$ | $CH_3$ | | |
| 601 | $2\text{-}CH_3,4\text{-}Cl\text{-}C_6H_3\text{-}O\text{-}$ | $-(CH_2)_3-$ | $CH_3$ | $CH_3$ | | |
| 602 | $4\text{-}C_2H_5\text{-}C_6H_4\text{-}O\text{-}$ | $-(CH_2)_3-$ | $CH_3$ | $CH_3$ | | |
| 603 | $4\text{-}iso\text{-}C_3H_7\text{-}C_6H_4\text{-}O\text{-}$ | $-(CH_2)_3-$ | $CH_3$ | $CH_3$ | | |
| 604 | $4\text{-}t\text{-}C_4H_9\text{-}C_6H_4\text{-}O\text{-}$ | $-(CH_2)_3-$ | $CH_3$ | $CH_3$ | | |
| 605 | $C_6H_5\text{-}O\text{-}$ | $-CH_2-CH(CH_3)-CH_2-$ | $CH_3$ | $CH_3$ | | |
| 606 | $C_6H_5\text{-}O\text{-}$ | $-(CH_2)_4-$ | $CH_3$ | $CH_3$ | | |
| 607 | $2\text{-}Cl\text{-}C_6H_4\text{-}O\text{-}$ | $-(CH_2)_4-$ | $CH_3$ | $CH_3$ | | |
| 608 | $4\text{-}Cl\text{-}C_6H_4\text{-}O\text{-}$ | $-(CH_2)_4-$ | $CH_3$ | $CH_3$ | | |
| 609 | $2,4\text{-}Cl_2\text{-}C_6H_3\text{-}O\text{-}$ | $-(CH_2)_4-$ | $CH_3$ | $CH_3$ | | |
| 610 | $2,6\text{-}Cl_2\text{-}C_6H_3\text{-}O\text{-}$ | $-(CH_2)_4-$ | $CH_3$ | $CH_3$ | | |
| 611 | $2\text{-}CH_3\text{-}C_6H_4\text{-}O\text{-}$ | $-(CH_2)_4-$ | $CH_3$ | $CH_3$ | | |
| 612 | $4\text{-}CH_3\text{-}C_6H_4\text{-}O\text{-}$ | $-(CH_2)_4-$ | $CH_3$ | $CH_3$ | | |
| 613 | $C_6H_5\text{-}O\text{-}$ | $-CH_2-CH_2-CH(CH_3)-CH_2-$ | $CH_3$ | $CH_3$ | | |
| 614 | $C_6H_5\text{-}O\text{-}$ | $-(CH_2)_5-$ | $CH_3$ | $CH_3$ | | |
| 615 | $3\text{-}Cl\text{-}C_6H_4\text{-}O\text{-}$ | $-(CH_2)_5-$ | $CH_3$ | $CH_3$ | | |
| 616 | $C_6H_5\text{-}O\text{-}$ | $-(CH_2)_3-CH(CH_3)-CH_2-$ | $CH_3$ | $CH_3$ | | |
| 617 | $C_6H_5\text{-}O\text{-}$ | $-(CH_2)_6-$ | $CH_3$ | $CH_3$ | | |
| 618 | $3\text{-}Cl\text{-}C_6H_5\text{-}O\text{-}$ | $-(CH_2)_6-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 619 | $C_6H_5$-O- | $-(CH_2)_4-CH(CH_3)-CH_2-$ | $CH_3$ | $CH_3$ | | |
| 620 | A 1*) | - | $CH_3$ | $CH_3$ | | |
| 621 | A 2*) | - | $CH_3$ | $CH_3$ | Öl | 2955,1721,1434,1243,1176,755 |
| 622 | A 3*) | - | $CH_3$ | $CH_3$ | | |
| 623 | A 4*) | - | $CH_3$ | $CH_3$ | | |
| 624 | A 5*) | - | $CH_3$ | $CH_3$ | | |
| 625 | A 6*) | - | $CH_3$ | $CH_3$ | | |
| 626 | A 7*) | - | $CH_3$ | $CH_3$ | | |
| 627 | A 8*) | - | $CH_3$ | $CH_3$ | | |
| 628 | A 9*) | - | $CH_3$ | $CH_3$ | | |
| 629 | A 10*) | - | $CH_3$ | $CH_3$ | | |
| 630 | A 11*) | - | $CH_3$ | $CH_3$ | | |
| 631 | A 12*) | - | $CH_3$ | $CH_3$ | | |
| 632 | A 13*) | - | $CH_3$ | $CH_3$ | | |
| 633 | A 14*) | - | $CH_3$ | $CH_3$ | | |
| 634 | A 15*) | - | $CH_3$ | $CH_3$ | | |
| 635 | A 15*) | - | $CH_3$ | $CH_3$ | | |
| 636 | A 17*) | - | $CH_3$ | $CH_3$ | | |
| 637 | N-Pyrrolyl | -CH(iso-$C_3H_7$)- | $CH_3$ | $CH_3$ | | |
| 638 | 4-tert.-Butyl-$C_6H_4$ | $-CH_2-C(CH_3)=CH-CH=CH-$ | $CH_3$ | $CH_3$ | | |
| 639 | H | $-CH_2-CH(CH_3)-CH_2-CH(CH_3)-$ | $CH_3$ | $CH_3$ | | |
| 640 | H | $-CH_2-CH(CH_3)-CH_2-CH(C_2H_5)-$ | $CH_3$ | $CH_3$ | | |
| 641 | H | $-CH_2-CH(CH_3)-CH_2-CH(n-C_3H_7)-$ | $CH_3$ | $CH_3$ | | |
| 642 | H | $-CH_2-CH(CH_3)-CH_2-CH(i-C_3H_7)-$ | $CH_3$ | $CH_3$ | | |

Tabelle 1 (Fortsetzung)

| Nr. | $R^3$ | $(X)_n$ | $R^2$ | $R^1$ | Fp (°C) | IR ($cm^{-1}$) |
|---|---|---|---|---|---|---|
| 643 | H | $-CH_2-C(CH_3)_2-CH_2-CH(CH_3)-CH_2-$ | $CH_3$ | $CH_3$ | | |
| 644 | H | $-(CH_2)_5-CH(C_2H_5)-$ | $CH_3$ | $CH_3$ | | |
| 645 | H | $-(CH_2)_5-CH(n-C_3H_7)-$ | $CH_3$ | $CH_3$ | | |
| 646 | H | $-(CH_2)_4-O-CH_2-C(CH_3)_2-$ | $CH_3$ | $CH_3$ | | |
| 647 | H | $-CH_2-O-CH_2-C(CH_3)_2-$ | $CH_3$ | $CH_3$ | | |
| 648 | $C_6H_5$ | $-CH=CH-(CH_2)_4-$ | $CH_3$ | $CH_3$ | | |
| 649 | H | $-CH_2-C(CH_3)_2-CH_2-CH(CH_3)_2-CH_2-$ | $CH_3$ | $CH_3$ | | |
| 650 | H | $-CH_2-CH(CH_3)-(CH_2)_2-CH(i-C_3H_7)-$ | $CH_3$ | $CH_3$ | | |
| 651 | 1-Methylcyclopropyl | - | $CH_3$ | $CH_3$ | Öl | 2967,1720, 1322,1243, 1156,755 |
| 652 | 2-Methylcyclopropyl | - | $CH_3$ | $CH_3$ | | |
| 653 | 2-Phenylcyclopropyl | - | $CH_3$ | $CH_3$ | | |
| 654 | 1-Methylcyclohexyl | - | $CH_3$ | $CH_3$ | | |
| 655 | $2-Cl-C_6H_4$ | $-CHCl-$ | $CH_3$ | $CH_3$ | | |
| 656 | 1-Methylcyclopropyl | - | $CH_3$ | $C_2H_5$ | | |
| 657 | 1-Methylcyclopropyl | - | $CH_3$ | $i-C_3H_7$ | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Ver teilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Einige der neuen Verbindungen haben sehr gute Wirksamkeit gegen humanpathogene Pilze, wie Trichophyton mentagrophytes und Candida albicans.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 286 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 286 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungs produktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 286 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 286 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 286 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 286 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 286 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 286 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 286 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,

N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

## Anwendungsbeispiele

Als Vergleichswirkstoffe wurden N-Tridecyl-2,6-dimethylmorpholin (A) - bekannt aus DE 1 164 152 - und die Verbindung

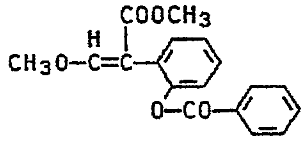

(B)

- bekannt aus EP-178 826 - benutzt.

Anwendungsbeispiel 1

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Abtrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 286 und 316 bei der Anwendung als 0,025 %ige ( Gew.%) Spritzbrühe eine bessere fungizide Wirkung zeigen (90 %) als die bekannten Vergleichswirkstoffe A (35 %) und B (35 %).

Anwendungsbeispiel 2

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Spoorensuspension von Pyricularia oryzae inoculiert. Anschließend wurden die Versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

Das Ergebnis zeigt, daß die Wirkstoffe 286, 83, 178 und 513 bei der Anwendung als 0,05 %ige Spritzbrühe eine bessere fungizide Wirkung (97 %) zeigen als der bekannte Vergleichswirkstoff A (55 %).

**Patentansprüche**

1. Ortho-substituierte Carbonsäurebenzylester der allgemeinen Formel I,

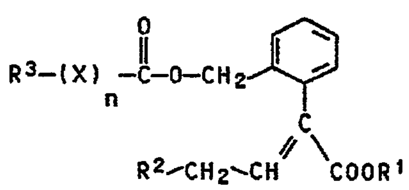

(I)

in der

$R^1$ $C_1$-$C_5$-Alkyl,

$R^2$ Wasserstoff, $C_1$-$C_4$-Alkyl, $C_1$-$C_4$-Alkoxy

$R^3$ Wasserstoff, Halogen, Cyano, Aryl, Aryloxy, wobei der aromatische Ring gegebenenfalls durch einen oder mehrere der folgenden Reste substituiert ist:
$C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl, Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ Heteroaryl, Adamantyl, Fluorenyl oder einen $C_3$-$C_7$-Cycloalkylrest oder $C_5$-$C_6$-Cycloalkenylrest bedeutet, wobei die Reste gegebenenfalls substituiert sind durch $C_1$-$C_4$-Alkyl, Methyl, Ethyl, Halogen $C_1$-$C_2$-Halogenalkyl, $C_3$-$C_4$-Alkenyl $C_2$-$C_4$-Halogenalkenyl, Methoxycarbonyl-$C_3$-$C_4$-Alkenyl, Cyclopentylidenmethyl, Halogenphenyl $C_1$-$C_2$-Alkoxyphenyl, $C_1$-$C_4$-Alkylphenyl,

X einen, gegebenenfalls durch Halogen oder Hydroxy sustituierten, gegebenenfalls ungesättigten $C_1$-$C_{12}$-Alkylenrest bedeutet und

n die Zahlen 0 oder 1 bedeutet.

2. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge einer Verbindung der Formel I

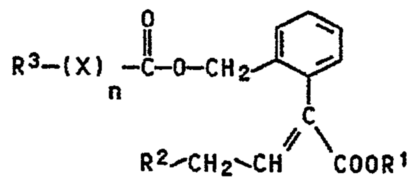

(I)

in der
$R^1$ $C_1$-$C_5$-Alkyl,
$R^2$ Wasserstoff, $C_1$-$C_4$-Alkyl, $C_1$-$C_4$-Alkoxy
$R^3$ Wasserstoff, Halogen, Cyano, Aryl, Aryloxy, wobei der aromatische Ring gegebenenfalls durch einen oder mehrere der folgenden Reste substituiert ist:
$C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl, Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ Heteroaryl, Adamantyl, Fluorenyl oder einen $C_3$-$C_7$-Cycloalkylrest oder $C_5$-$C_6$-Cycloalkenylrest bedeutet, wobei die Reste gegebenenfalls substituiert sind durch $C_1$-$C_4$-Alkyl, Methyl, Ethyl, Halogen $C_1$-$C_2$-Halogenalkyl, $C_3$-$C_4$-Alkenyl $C_2$-$C_4$-Halogenalkenyl, Methoxycarbonyl-$C_3$-$C_4$-Alkenyl, Cyclopentylidenmethyl, Halogenphenyl $C_1$-$C_2$-Alkoxyphenyl, $C_1$-$C_4$-Alkylphenyl,
X einen, gegebenenfalls durch Halogen oder Hydroxy sustituierten, gegebenenfalls ungesättigten $C_1$-$C_{12}$-Alkylenrest bedeutet und
n die Zahlen 0 oder 1 bedeutet, behandelt.

3. Fungizid, enthaltend einen inerten Trägerstoff und eine fungizid wirksame Menge einer Verbindung der Formel I

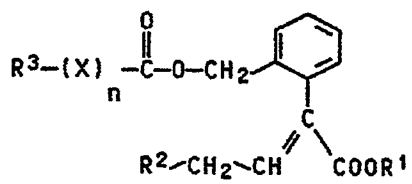

(I)

in der
$R^1$ $C_1$-$C_5$-Alkyl,
$R^2$ Wasserstoff, $C_1$-$C_4$-Alkyl, $C_1$-$C_4$-Alkoxy
$R^3$ Wasserstoff, Halogen, Cyano, Aryl, Aryloxy, wobei der aromatische Ring gegebenenfalls durch einen oder mehrere der folgenden Reste substituiert ist:
$C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl, Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ Heteroaryl, Adamantyl, Fluorenyl oder einen $C_3$-$C_7$-Cycloalkylrest oder $C_5$-$C_6$-Cycloalkenylrest bedeutet, wobei die Reste gegebenenfalls substituiert sind durch $C_1$-$C_4$-Alkyl, Methyl, Ethyl, Halogen $C_1$-$C_2$-Halogenalkyl, $C_3$-$C_4$-Alkenyl $C_2$-$C_4$-Halogenalkenyl, Methoxycarbonyl-$C_3$-$C_4$-Alkenyl, Cyclopentylidenmethyl, Halogenphenyl $C_1$-$C_2$-Alkoxyphenyl, $C_1$-$C_4$-Alkylphenyl,
X einen, gegebenenfalls durch Halogen oder Hydroxy sustituierten, gegebenenfalls ungesättigten $C_1$-$C_{12}$-Alkylenrest bedeutet und
n die Zahlen 0 oder 1 bedeutet.

4. Verfahren zur Herstellung eines fungiziden Mittels, dadurch gekennzeichnet, daß man eine oder mehrere Verbindungen der Formel I gemäß Anspruch 1 mit einem festen oder flüssigen Trägerstoff sowie gegebenenfalls mit einem oder mehreren oberflächenaktiven Mitteln mischt.

5. Phenylglyoxylsäureester der Formel VI

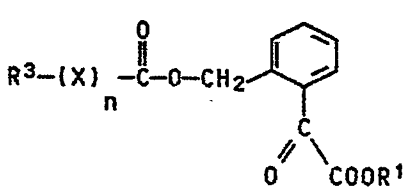

(VI)

in der
$R^1$ $C_1$-$C_5$-Alkyl,
$R^3$ Wasserstoff, Halogen, Cyano, Aryl, Aryloxy, wobei der aromatische Ring gegebenenfalls durch einen oder mehrere der folgenden Reste substituiert ist:
$C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl-Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ Heteroaryl, Adamantyl, Fluorenyl, oder einen $C_3$-$C_7$-Cycloalkylrest oder $C_5$-$C_6$-Cyclo alkenylrest bedeutet, wobei die Reste gegebenenfalls substituiert sind durch $C_1$-$C_4$-Alkyl, Methyl, Ethyl, Halogen, $C_1$-$C_2$-Halogenalkyl, $C_3$-$C_4$-Alkenyl, $C_2$-$C_4$-Halogenalkenyl, Methoxycarbonyl-$C_3$-$C_4$-Alkenyl, Cyclopentylidenmethyl, Halogenphenyl, $C_1$-$C_2$-Alkoxyphenyl, $C_1$-$C_4$-Alkylphenyl,
X einen, gegebenenfalls durch Halogen oder Hydroxy substituierten, gegebenenfalls ungesättigten C1-C12-Alkylenrest bedeutet und
n die Zahlen 0 oder 1 bedeutet.

6. Ortho-[2,2-Dimethyl-3(2′,2′-dichlorvinyl)-cyclopropylcarboxymethylen]-phenylglyoxylsäuremethylester.

7. α-[ortho-(2,2-Dimethyl-3(2′,2′-dichlorvinyl)-cyclopropyl-carboxymethylen)-phenyl]-β-methylacrylsäuremethylester.

8. α-[ortho-(2,2-Dimethyl-3(2′,2′-dichlorvinyl)-cyclopropylcarboxymethylen)-phenyl]-β-ethylacrylsäuremethylester.

9. α-[ortho-(1-Methylcyclopropylcarboxymethylen)-phenyl]-β-ethyl-acrylsäuremethylester.

**Claims**

1. An ortho-substituted benzyl carboxylate of the formula I

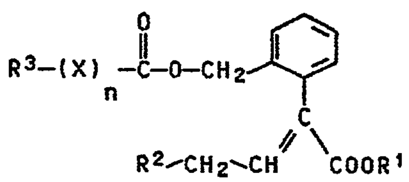
(I)

where
$R^1$ is $C_1$–$C_5$-alkyl,
$R^2$ is hydrogen, $C_1$–$C_4$-alkyl or $C_1$–$C_4$-alkoxy,
$R^3$ is hydrogen, halogen, cyano, aryl or aryloxy, the aromatic ring being unsubstituted or substituted by one or more of the following radicals:
$C_1$–$C_6$-alkyl, $C_2$–$C_4$-alkenyl, $C_1$–$C_2$-haloalkyl, $C_1$–$C_6$-alkoxy, $C_1$–$C_4$-alkoxy-$C_1$–$C_4$-alkyl, aryl, aryl-$C_1$–$C_2$-alkyl, aryloxy, aryloxy-$C_1$–$C_4$-alkyl, aryloxy-$C_1$–$C_4$-alkoxy, haloaryloxy-$C_1$–$C_4$-alkoxy, halogen, halo-$C_1$–$C_4$-alkoxy, $C_1$–$C_4$-alkylthio, thiocyanato, cyano and nitro, or $R^3$ is hetaryl, adamantyl, fluorenyl or $C_3$–$C_7$-cycloalkyl or $C_5$–$C_6$-cycloalkenyl, the radicals being unsubstituted or substituted by $C_1$–$C_4$-alkyl, methyl, ethyl, halogen, $C_1$–$C_4$-haloalkyl, $C_3$–$C_4$-alkenyl, $C_2$–$C_4$-haloalkenyl, methoxycarbonyl-$C_3$–$C_4$-alkenyl, cyclopentylidenemthyl, halophenyl, $C_1$–$C_2$-alkoxyphenyl or $C_1$–$C_4$-alkylphenyl,
X is saturated or unsaturated $C_1$–$C_{12}$-alkylene which is unsubstituted or substituted by halogen or hydroxyl, and
n is 0 or 1.

2. A method for controlling fungi, wherein the fungi or the materials, plants, seed or the soil threatened by fungal attack are treated with a fungicidally effective
amount of a compound of the formula I

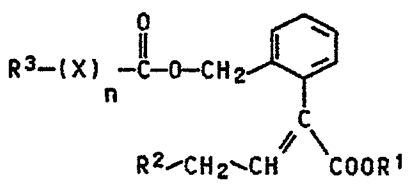
(I)

where
$R^1$ is $C_1$–$C_5$-alkyl,
$R^2$ is hydrogen, $C_1$–$C_4$-alkyl or $C_1$–$C_4$-alkoxy,
$R^3$ is hyrogen, halogen, cyano, aryl or aryloxy, the aromatic ring being unsubstituted or substituted by one or more of the following radicals: $C_1$–$C_6$-alkyl, $C_2$–$C_4$-alkenyl, $C_1$–$C_2$-haloalkyl, $C_1$–$C_6$-alkoxy, $C_1$–

$C_4$-alkoxy-$C_1$–$C_4$-alkyl, aryl, aryl-$C_1$–$C_2$-alkyl, aryloxy, aryloxy-$C_1$–$C_4$-alkyl, aryloxy-$C_1$–$C_4$-alkoxy, haloaryloxy-$C_1$–$C_4$-alkoxy, halogen, halo-$C_1$–$C_4$-alkoxy, $C_1$–$C_4$-alkylthio, thiocyanato, cyano and nitro, or $R^3$ is hetaryl, adamantyl, fluorenyl or $C_3$–$C_7$-cycloalkyl or $C_5$–$C_6$-cycloalkenyl, the radicals being unsubstituted or substituted by $C_1$–$C_4$-alkyl, methyl, ethyl, halogen, $C_1$–$C_2$-haloalkyl, $C_3$–$C_4$-alkenyl, $C_2$–$C_4$-haloalkenyl, methoxycarbonyl-$C_3$–$C_4$-alkenyl, cyclopentylidenemethyl, halophenyl, $C_1$–$C_2$-alkoxyphenyl or $C_1$–$C_4$-alkylphenyl,

X is saturated or unsaturated $C_1$–$C_{12}$-alkylene which is unsubstituted or substituted by halogen or hydroxyl, and

n is 0 or 1.

3. A fungicide containing an inert carrier and a fungicidally effective amount of the formula I

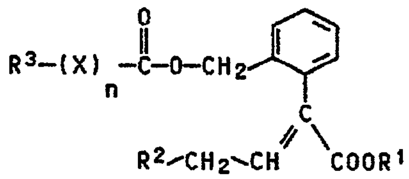

(I)

where

$R^1$ is $C_1$–$C_5$-alkyl,

$R^2$ is hydrogen, $C_1$–$C_4$-alkyl or $C_1$–$C_4$-alkoxy,

$R^3$ is hydrogen, halogen, cyano, aryl or aryloxy, the aromatic ring being unsubstituted or substituted by one or more of the following radicals: $C_1$–$C_6$-alkyl, $C_2$–$C_4$-alkenyl, $C_1$–$C_2$-haloalkyl, $C_1$–$C_6$-alkoxy, $C_1$–$C_4$-alkoxy-$C_1$–$C_4$-alkyl, aryl, aryl-$C_1$–$C_2$-alkyl, aryloxy, aryloxy-$C_1$–$C_4$-alkyl, aryloxy-$C_1$–$C_4$-alkoxy, haloaryloxy-$C_1$–$C_4$-alkoxy, halogen, halo-$C_1$–$C_4$-alkoxy, $C_1$–$C_4$-alkylthio, thiocyanato, cyano and nitro, or $R^3$ is hetaryl, adamantyl, fluorenyl or $C_3$–$C_7$-cycloalkyl or $C_5$–$C_6$-cycloalkenyl, the radicals being unsubstituted or substituted by $C_1$–$C_4$-alkyl, methyl, ethyl, halogen, $C_1$–$C_2$-haloalkyl, $C_3$–$C_4$-alkenyl, $C_2$–$C_4$-haloalkenyl, methoxycarbonyl-$C_3$–$C_4$-alkenyl, cyclopentylidenemethyl, halophenyl, $C_1$–$C_2$-alkoxyphenyl or $C_1$–$C_4$-alkylphenyl,

X is saturated or unsaturated $C_1$–$C_{12}$-alkylene which is unsubstituted or substituted by halogen or hydroxyl, and

n is 0 or 1.

4. A process for preparing a fungicide, wherein one or more compounds of the formula I as claimed in claim 1 are mixed with a solid or liquid carrier and, optionally, with one or more surfactants.

5. A phenylglyoxylic ester of the formula VI

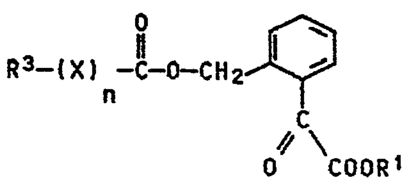

(VI)

where

$R^1$ is $C_1$–$C_5$-alkyl,

$R^3$ is hydrogen, halogen, cyano, aryl or aryloxy, the aromatic ring being unsubstituted or substituted by one or more of the following radicals: $C_1$–$C_6$-alkyl, $C_2$–$C_4$-alkenyl, $C_1$–$C_2$-haloalkyl, $C_1$–$C_6$-alkoxy, $C_1$–$C_4$-alkoxy-$C_1$–$C_4$-alkyl, aryl, aryl-$C_1$–$C_2$-alkyl, aryloxy, aryloxy-$C_1$–$C_4$-alkyl, aryloxy-$C_1$–$C_4$-alkoxy, haloaryloxy-$C_1$–$C_4$-alkoxy, halogen, halo-$C_1$–$C_4$-alkoxy, $C_1$–$C_4$-alkylthio, thiocyanato, cyano and nitro, or $R^3$ is hetaryl, adamantyl, fluorenyl or $C_3$–$C_7$-cycloalkyl or $C_5$–$C_6$-cycloalkenyl, the radicals being unsubstituted or substituted by $C_1$–$C_4$-alkyl, methyl, ethyl, halogen, $C_1$–$C_2$-haloalkyl, $C_3$–$C_4$-alkenyl, $C_2$–$C_4$-haloalkenyl, methoxycarbonyl-$C_3$–$C_4$-alkenyl, cyclopentylidenemetyhl, halophenyl, $C_1$–$C_2$-alkoxyphenyl or $C_1$–$C_4$-alkylphenyl,

X is saturated or unsaturated $C_1$–$C_{12}$-alkylene which is unsubstituted or substituted by halogen or hydroxyl, and

n is 0 or 1.

6. Methyl ortho-[2,2-dimethyl-3-(2′,2′-dichlorovinyl)-cyclopropylcarboxymethylene]-phenylglyoxylate.

7. Methyl α-[ortho-(2,2-dimethyl-3-(2′,2′-dichlorovinyl)-cyclopropylcarboxymethylene)-phenyl]-β-methylacrylate.

8. Methyl α-[ortho-(2,2-dimethyl-3-(2′,2′-dichlorovinyl)-cyclopropylcarboxymethylene)-phenyl]-β-ethylacrylate.

9. Methyl α-[ortho-(1-methylcyclopropylcarboxy-methylene)-phenyl]-β-ethylacrylate.

## Revendications

1. Esters benzyliques d'acides carboxyliques substitués en position ortho, de la formule générale I

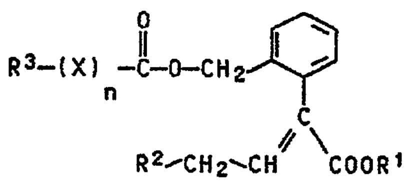

(I)

dans laquelle
$R^1$ représente un radical alkyle en $C_1$–$C_5$,
$R^2$ représente un atome d'hydrogène, un radical alkyle en $C_1$–$C_4$, alcoxy en $C_1$–$C_4$,
$R^3$ représente un atome d'hydrogène, un atome d'halogène, un radical cyano, aryle, aryloxy, où le noyau aromatique est éventuellement substitué par un ou plusieurs des groupes suivants: alkyle en $C_1$–$C_6$, alcényle en $C_2$–$C_4$, halogénoalkyle en $C_1$–$C_2$, alcoxy en $C_1$–$C_6$, alcoxy ($C_1$–$C_4$)-alkyle en $C_1$–$C_4$, aryle, aryl-alkyle en $C_1$–$C_4$, aryloxy, aryloxy-alkyle en $C_1$–$C_4$, aryloxy-alcoxy en $C_1$–$C_4$, halogénoaryloxy-alcoxy en $C_1$–$C_4$, halogéno, halogéno-alcoxy en $C_{11}$–$C_4$, alkylthio en $C_1$–$C_4$, thiocyanato, cyano, nitro, ou bien $R^3$ représente un groupe hétéroaryle, adamantyle, fluorényle, ou un groupe cycloalkyle en $C_3$–$C_7$ ou cycloalcényle en $C_5$–$C_6$, où les groupes peuvent éventuellement être substitués par des radicaux alkyle en $C_1$–$C_4$, méthyle, éthyle, des atomes d'halogènes, des radicaux halogénoalkyle en $C_1$–$C_2$, alcényle en $C_3$–$C_4$, halogénoalcényle en $C_2$–$C_4$, méthoxycarbonylalcényle en $C_3$–$C_4$, cyclopentylidène méthyle, halogénophényle, alcoxy($C_1$–$C_2$)-phényle, alkyl($C_1$–$C_4$)-phényle.
X représente un groupe alkylène en $C_1$–$C_{12}$ à substitution halogénée ou hydroxylique, éventuellement insaturé et
n est égal à 0 ou à 1.

2. Procédé de lutte contre des champignons, caractérisé en ce que l'on traite les champignons, le sol, les semences, les plantes ou les matières risquant d'être attaquées par des champignons, par une quantité active du point de vue fongicide d'un composé de la formule I

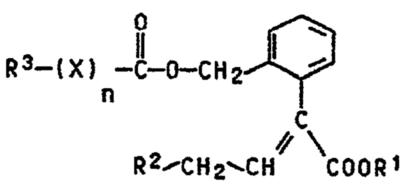

(I)

dans laquelle
$R^1$ représente un radical alkyle en $C_1$–$C_5$,
$R^2$ représente un atome d'hydrogène, un radical alkyle en $C_1$–$C_4$, alcoxy en $C_1$–$C_4$,
$R^3$ représente un atome d'hydrogène, un atome d'halogène, un radical cyano, aryle, aryloxy, où le noyau aromatique est éventuellement substitué par un ou plusieurs des groupes suivants: alkyle en $C_1$–$C_6$, alcényle en $C_2$–$C_4$, halogénoalkyle en $C_1$–$C_2$, alcoxy en $C_1$–$C_6$, alcoxy($C_1$–$C_4$)-alkyle en $C_1$–$C_4$, aryle, aryl-alkyle en $C_1$–$C_2$, aryloxy, aryloxy-alkyle en $C_1$–$C_4$, aryloxy-alcoxy en $C_1$–$C_4$, halogénoaryloxy-alcoxy en $C_1$–$C_4$, halogéno, halogéno-alcoxy en $C_1$–$C_4$, alkylthio en $C_1$–$C_4$, thiocyanato, cyano, nitro, ou bien $R^3$ représente un groupe hétéroaryle, adamantyle, fluorényle, ou un groupe cycloalkyle en $C_3$–$C_7$ ou cycloalcényle en $C_5$–$C_6$, où les groupes peuvent éventuellement être substitués par des radicaux alkyle en $C_1$–$C_4$, méthyle, éthyle, des atomes d'halogènes, des radicaux halogénoalkyle en $C_1$–$C_2$, alcényle en $C_3$–$C_4$, halogénoalcényle en $C_2$–$C_4$, méthoxycarbonylalcényle en $C_3$–$C_4$, cyclopentylidène méthyle, halogénophènyle, alcoxy($C_1$–$C_2$)-phényle, alkyl($C_1$–$C_4$)-phényle.
X représente un groupe alkylène en $C_1$–$C_{12}$ à substitution halogénée ou hydroxylique, éventuellement insaturé et
n est égal à 0 ou à 1.

3. Fongicide, contenant un support ou véhicule inerte et une proportion active du point de vue fongicide d'un composé de la formule I

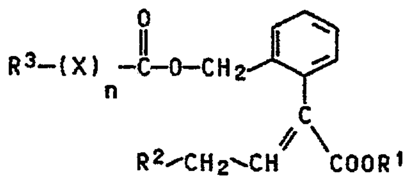

(I)

dans laquelle
$R^1$ représente un radical alkyle en $C_1$–$C_5$,
$R^2$ représente un atome d'hydrogène, un radical alkyle en $C_1$–$C_4$, alcoxy en $C_1$–$C_4$,
$R^3$ représente un atome d'hydrogène, un atome d'halogène, un radical cyano, aryle, aryle, aryloxy, où le noyau aromatique est éventuellement substitué par un ou plusieurs des groupes suivants: alkyle en $C_1$–$C_6$, alcényle en $C_2$–$C_4$, halogénoalkyle en $C_1$–$C_2$, alcoxy en $C_1$–$C_6$, alcoxy($C_1$–$C_4$)-alkyle en $C_1$–$C_4$, aryle, aryl-alkyle en $C_1$–$C_2$, aryloxy, aryloxy-alkyle en $C_1$–$C_4$, aryloxy-alcoxy en $C_1$–$C_4$, halogéno-aryloxy-alcoxy en $C_1$–$C_4$, halogéno, halogéno-alcoxy en $C_1$–$C_4$, alkylthio en $C_1$–$C_4$, thiocyanato, cyano, nitro, ou bien $R^3$ représente un groupe hétéroaryle, adamantyle, fluorényle, ou un groupe cycloalkyle en $C_3$–$C_7$ ou cycloalcényle en $C_5$–$C_6$, où les groupes peuvent éventuellement être substitués par des radicaux alkyle en $C_1$–$C_4$, méthyle, éthyle, des atomes d'halogènes, des radicaux halogénoalkyle en $C_1$–$C_2$, alcényle en $C_3$–$C_4$, halogénoalcényle en $C_2$–$C_4$, méthoxycarbonyl-alcényle en $C_3$–$C_4$, cyclopentylidène méthyle, halogénophényle, alcoxy($C_1$–$C_2$)-phényle, alkyl($C_1$–$C_4$)-phényle.
X représente un groupe alkylène en $C_1$–$C_{12}$ à substitution halogénée ou hydroxylique, éventuellement insaturé et
n est égal à 0 ou à 1.

4. Procédé de préparation d'un composition ou d'un agent fongicide, caractérisé en ce que l'on mélange un ou plusieurs composés de la formule I suivant la revendication 1, un véhicule ou support solide ou liquide, comme éventuellement aussi à un ou plusieurs agents surfactifs ou tensio-actifs.

5. Esters de l'acide phénylglyoxylique de la formule VI

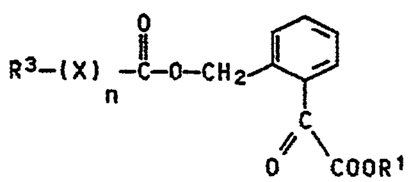

(VI)

dans laquelle
$R^1$ représente un radical alkyle en $C_1$–$C_5$,
$R^3$ représente un atome d'hydrogène, un atome d'halogène, un radical cyano, aryle, aryloxy, où le noyau aromatique est éventuellement substitué par un ou plusieurs des groupes suivants: alkyle en $C_1$–$C_6$, alcényle en $C_2$–$C_4$, halogénoalkyle en $C_1$–$C_2$, alcoxy en $C_1$–$C_6$, alcoxy($C_1$–$C_4$)-alkyle en $C_1$–$C_4$, aryle, aryl-alkyle en $C_1$–$C_2$, aryloxy, aryloxy-alkyle en $C_1$–$C_4$, aryloxy-alcoxy en $C_1$–$C_4$, halogénoaryloxy-alcoxy en $C_1$–$C_4$, halogéno, halogéno-alcoxy en $C_1$–$C_4$, alkylthio en $C_1$–$C_4$, thiocyanato, cyano, nitro, ou bien $R^3$ représente un groupe hétéroaryle, adamantyle, fluorényle, ou un groupe cycloalkyle en $C_3$–$C_7$ ou cycloalcényle en $C_5$–$C_6$, ou les groupes sont éventuellement substitués par des radicaux alkyle en $C_1$–$C_4$, méthyle, éthyle, des atomes d'halogènes, des radicaux halogénoalkyle en $C_1$–$C_2$, alcényle en $C_3$–$C_4$, halogénoalcényle en $C_2$–$C_4$, cyclopentylidène méthyle, halogénophényle, alcoxy($C_1$–$C_2$)-phényle, alkyl($C_1$–$C_4$)-phényle,
X représente un groupe alkylène en $C_1$–$C_2$ à substitution halogénée ou hydroxylique, éventuellement insaturé et
n est égal à 0 ou à 1.

6. Ortho-[2,2-diméthyl-3(2′, 2′-dichlorovinyl)-cyclopropylcarboxyméthylène]-phénylglyoxyate de méthyle.

7. α-[Ortho-2,2-diméthyl-3(2′,2′-dichlorovinyl)-cyclopropyl-carboxyméthylène)-phényl]-β-méthyl-acrylate de méthyle.

8. α-[Ortho-(2,2-diméthyl-3(2′,2′-dichlorovinyl)-cyclopropylcarboxyméthylène)-phényl]-β-éthyl-acrylate de méthyle.

9. α-[Ortho-(1-méthylcyclopropylcarboxyméthylène)-phényl]-β-éthyl-acrylate de méthyle.